# EUROPEAN PATENT APPLICATION

(11) **EP 3 342 383 A1**
(43) Date of publication of application: **04.07.2018**
(21) Application number: 16841179.1
(22) Date of filing: 05.04.2016
(51) Int. Cl.: A61F 13/15

(54) **METHOD AND DEVICE FOR MANUFACTURING SHEET-SHAPED MEMBER FOR ABSORBENT ARTICLE**

(30) Priority: 28.08.2015 JP 2015168523
(71) Applicant: Unicharm Corporation, Shikokuchuo-shi, Ehime 799-0111 (JP)
(72) Inventor: TAKEUCHI, Kenji, Kanonji-shi Kagawa 769-1602 (JP)
(74) Representative: Peter, Julian
(86) International application number: PCT/JP2016/061063
(87) International publication number: WO 2017/038147

(57) **Abstract**

Provided is a method for manufacturing a sheet-like member of an absorbent article by stacking and fixing a first sheet (3a) and a second sheet (5a) at a merging position (Pj). The first sheet (3a) has a first pitch in a direction in which the first sheet (3a) continues. On the second sheet (5a), a display item (IL) is formed at a second pitch before the second sheet (5a) becomes in a state of a sheet roll (5ar), and the second pitch is smaller than the first pitch. The second sheet (5a) is wrapped around three rolls (31u, 31m, 31d) located upstream from a device (51). A sensor (31s) outputs a value depending on force that is applied to a center roll (31m) by the second sheet (5a). Based on the value, a device (21) feeds the second sheet (5a) from the sheet roll (5ar) . The device (51) sends-off the second sheet (5a) toward the merging position (Pj) while changing the stretching state of the second sheet (5a) so that the display items (IL) are aligned at the first pitch and so that the display item (IL) is located at a target position on the first sheet (5a) is determined based on the first pitch.

## Description

### [Technical Field]

The present invention relates to a method and an apparatus for manufacturing a sheet-like member associated with an absorbent article such as a disposable diaper.

### [Background Art]

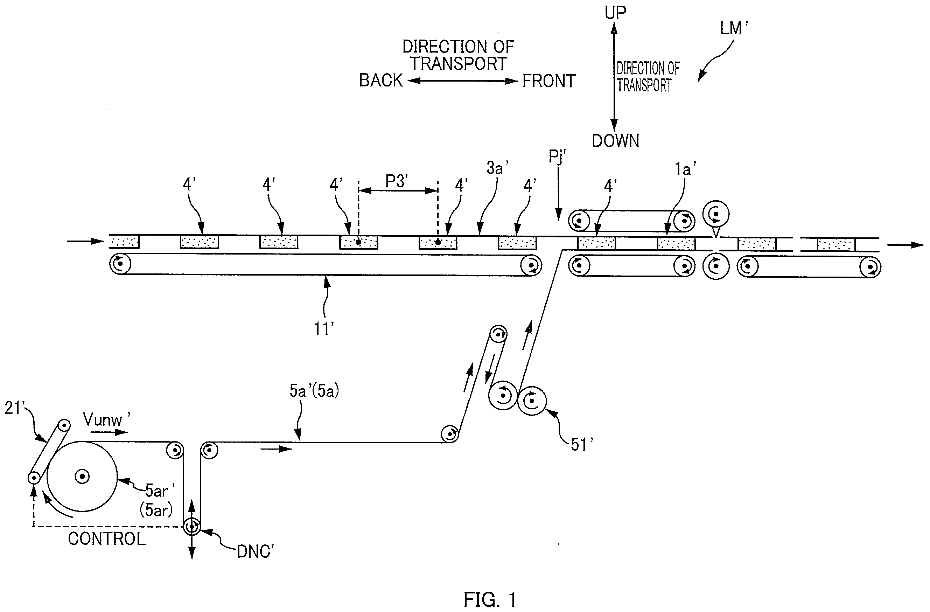

As shown in the schematic side view of Fig. 1, in a manufacturing line IM' of a disposable diaper exemplifying an absorbent article, a continuous first sheet 3a' and a continuous second sheet 5a' are stacked and fixed to manufacture a sheet-like member 1a'. PTL 1 discloses the following method as an example of a method for manufacturing such a sheet-like member 1a'.

First, the first sheet 3a' has a virtual predetermined product pitch P3' in the direction in which the first sheet 3a' continues, and the predetermined product pitch P3' is associated with a single piece of diaper. In this example, at this stage, absorbent bodies 4', 4' ... have already been fixed to the first sheet 3a' with being aligned at a product pitch P3' in the direction in which the first sheet 3a' continues. The first sheet 3a' in this state is transported by a transport device 11' in a direction of transport, which is the direction in which the first sheet 3a' continues.

The second sheet 5a' is basically installed to the manufacturing line IM' in a form of sheet roll 5ar'. But, before being wound into a form of the sheet roll 5ar', the second sheet 5a' has display items (e.g. illustrations; not shown) formed thereon at a print pitch P5' in the direction in which the second sheet 5a' continues; the print pitch P5' is smaller than the product pitch P3'. A feeding device 21' in the manufacturing line IM' feeds the second sheet 5a' from the sheet roll 5ar'. When a sending-off device 51' sends-off the fed second sheet 5a' toward the merging position Pj' in the transportation path of the first sheet 3a', the sending-off device 51' changes the stretching state of the second sheet 5a' so that display items of the second sheet 5a' are aligned at the product pitch P3', and so that the display items are located at target positions on the first sheet 3' which are determined based on the product pitch P3' and where the display items are to be placed. Thus, the second sheet 5a' are stacked and fixed onto the first sheet 3a' with the display items being appropriately placed according to the product pitch P3' of the first sheet 3a' .

But, in a manufacturing method disclosed in PTL 1, a so-called dancer roll DNC' is arranged at a position between the feeding device 21' and the sending-off device 51' in the transportation path of the second sheet 5a' . The second sheet 5a' is wrapped around the dancer roll DNC', and the tension of the second sheet 5a' during transportation is detected. Based on a detection signal, the feeding velocity Vunw' (m/sec) of the feeding device 21' is controlled. Thus, the tension of the second sheet 5a' keeps constant. The detail is as follow.

For example, if the tension of the second sheet 5a' fluctuates and becomes larger than half the weight of the dancer roll DNC', the dancer roll DNC' move upward higher than a predetermined position in such a manner that the dancer roll DNC' is pulled up by the second sheet 5a'. In this case, the feeding velocity Vunw' of the feeding device 21' increases so that the dancer roll DNC' move back to the predetermined position. Thereby, the tension is adjusted to the value before its fluctuation. On the contrary, if the tension fluctuates and becomes smaller than half the weight of the dancer roll DNC', the second sheet 5a' cannot support the weight of the dancer roll DNC' and the dancer roll DNC' moves downward lower than the predetermined position. In this case, the feeding velocity Vunw' of the feeding device 21' decreases so that the dancer roll DNC' move back to the predetermined position. Thereby, the tension is adjusted to the value before its fluctuation.

In the configuration including the dancer roll DNC' having such a function, considering a case where tension fluctuation of the second sheet 5a' which will be caused by changing the stretching state by the sending-off device 51' propagates backward upstream in the direction of transport. In this case, it is possible to reduce the fluctuation based on control of the foregoing feeding velocity Vunw'. This makes it possible to send the sending-off device 51' the second sheet 5a' whose tension fluctuation is suppressed. This also may contribute to improvement of accuracy for positioning display items at the target positions.

### [Citation List]

### [Patent Literature]

[PTL 1] Japanese Patent No. 4246020

### [Summary of Invention]

### [Technical Problem]

However, as mentioned above, based on reciprocating movement in a predetermined direction, the dancer roll DNC' detects tension fluctuation of the second sheet 5a'. That is, according to tension fluctuation, the dancer roll DNC' performs translational movement in the direction of reciprocating movement. This movement can cause the inertia-derived force of the dancer roll DNC' to act on the second sheet 5a', and consequently there is a possibility that another tension fluctuation of the second sheet 5a' occurs. For example, when the dancer roll DNC' in the stopping state starts to move, the foregoing inertia-derived force can act on the second sheet 5a' in a direction opposite the direction of movement. Consequently, there is a possibility that tension of the second sheet 5a' fluctuates. On the contrary, when the dancer roll DNC' in the moving state stops, the foregoing inertia-derived force can act on the second sheet 5a' in the direction of movement. Consequently, there is a possibility that tension of the second sheet 5a' fluctuates.

Thus, the second sheet 5a' whose tension fluctuation is not effectively suppressed is sent to the sending-off device 51'. This will reduce contribution to improvement of accuracy for positioning display items at the foregoing target positions. That is, there is a possibility to impair aligning the foregoing display items of the second sheet 5a' at the product pitch P3', etc. and to impair positioning with high accuracy the display items of the second sheet 5a' at target positions where the display items are placed on the first sheet 3a' .

The present invention has been made in view of the above described conventional problem, and an object thereof is to enable to stack and fix a second sheet onto a first sheet with high accuracy so that display items (e.g. illustrations) of the second sheet are aligned at a first pitch (e.g. a product pitch), and so that the display items are located at target positions on the first sheet, which is determined based on the first pitch and where display item is to be placed.

### [Solution to Problem]

In order to achieve an object described above, the main aspect of the present invention is
A method for manufacturing a sheet-like member associated with an absorbent article,
the manufacturing being performed by stacking and fixing a continuous first sheet and a continuous second sheet while aligning a direction in which the first sheet continues with a direction in which the second sheet continues,
the first sheet having a predetermined first pitch in the direction in which the first sheet continues,
   the first pitch being associated with a single piece of the absorbent article,
the second sheet having a display item at a second pitch in the direction in which the second sheet continues,
   the second pitch being smaller than the first pitch,
   the display item being formed before the second sheet becomes in a state of a sheet roll by being wound in the direction in which the second sheet continues,
the method including:
   feeding the second sheet by a feeding device from the sheet roll; and
   sending-off the second sheet by a sending-off device toward a merging position in a transportation path of the first sheet,
      the second sheet being fed and transported in a direction of transport,
      the direction of transport being the direction in which the second sheet continues,
   the feeding including:
      outputting a value with a first sensor; and
      performing by the feeding device based on the value a feeding operation of the second sheet from the sheet roll,
         the value depending on force that is applied to a center roll of three rolls by the second sheet wrapped around the three rolls,
         the three rolls being rotatably supported at a predetermined position upstream from the sending-off device in the direction of transport,
   the sending-off including
      changing by the sending-off device a stretching state of the second sheet in the direction of transport
      so that the display item of the second sheet being transported in the direction of transport is aligned at the first pitch, and
      so that the display item is located at a target position on the first sheet that is determined based on the first pitch and where the display item is to be placed.

Further,
A device for manufacturing a sheet-like member associated with an absorbent article,
the manufacturing being performed by stacking and fixing a continuous first sheet and a continuous second sheet while aligning a direction in which the first sheet continues with a direction in which the second sheet continues,
the first sheet having a predetermined first pitch in the direction in which the first sheet continues,
   the first pitch being associated with a single piece of the absorbent article,
   the second sheet having a display item at a second pitch in the direction in which the second sheet continues,
      the second pitch being smaller than the first pitch,
      the display item being formed before the second sheet becomes in a state of a sheet roll by being wound in the direction in which the second sheet continues,
   the device including:
      a feeding device that feeds the second sheet from the sheet roll;
      a sending-off device that sends-off the second sheet toward a merging position in a transportation path of the first sheet,
         the second sheet being fed and transported in in a direction of transport,
         the direction of transport being the direction in which the second sheet continues;
      three rolls that are rotatably supported at a predetermined position in the direction of transport, the predetermined position being between the feeding device and the sending-off device; and
      a first sensor that outputs a value,
         the value depending on force that is applied to a center roll of three rolls by the second sheet wrapped around the three rolls,
      based on the value, the feeding device performing feeding operation of the second sheet from the sheet roll,
      while sending-off the second sheet, the sending-off device changing a stretching state of the second sheet in the direction of transport
         so that the display item of the second sheet being transported in the direction of transport is aligned at the first pitch, and
         so that the display item is located at a target position on the first sheet that is determined based on the first pitch and where the display item is to be placed.

Features and objects of the present invention other than the above will become clear by the accompanying drawings and the description hereinbelow.

### [Advantageous Effects of Invention]

According to the present invention, it is possible to stack and fix a second sheet onto a first sheet with high accuracy so that display items (e.g. illustrations) of the second sheet are aligned at a first pitch (e.g. a product pitch), and so that the display items are located at target positions on the first sheet, which is determined based on the first pitch and where display item is to be placed.

### [Brief Description of Drawings]

[Fig. 1] Fig. 1 is a diagram illustrating an example of a method for manufacturing a sheet-like member 1a' associated with an absorbent article.
[Fig. 2] Fig. 2A is a schematic plan view of an absorbent main body 1 viewed from the non-skin side, and Fig. 2B is a cross-sectional view taken along line B-B of Fig. 2A.
[Fig. 3] Fig. 3 is a schematic plan view of the continuous body 1a of the absorbent main body which is to be the absorbent main body 1.
[Fig. 4] Fig. 4 is a schematic side view of a manufacturing line IM of a sheet-like member 1a according to the present embodiment.
[Fig. 5] Fig. 5 is a schematic magnified side view of a feeding device 21 and a tension-measuring device 31.
[Fig. 6] Fig. 6 is a schematic plan view of the continuous sheet 5a of a back sheet in which, in another factory or place, a plurality of illustrations IL, IL ... are printed by a print pitch P5 in a direction in which the sheet 5a continues.
[Fig. 7] Fig. 7 is a schematic plan view of the continuous sheet 5a of the back sheet in which an illustration ILa is printed in a narrow region.
[Fig. 8] Fig. 8A is a schematic plan view of an upstream part, in the direction of transport, of the processing system L5a of the continuous sheet 5a of the back sheet according to the present embodiment. Fig. 8B is a schematic plan view of the upstream part of the processing system L5a in which the devices 21 and 31 are arranged in a different manner from the foregoing embodiment.
[Fig. 9] Fig. 9 is a schematic side view of a manufacturing line IM according to the first modified example of the present embodiment.
[Fig. 10] Fig. 10 is a schematic side view of a manufacturing line LM according to the second modified example of the present embodiment.
[Fig. 11] Fig. 11 is a schematic side view of a manufacturing line LM according to the third modified example of the present embodiment.
[Fig. 12] Figs. 12A to 12E are schematic side views for illustrating a joining process of the third modified example.
[Fig. 13] Fig. 13 is a diagram illustrating two positions Paj and Pajs which should be positioned in the joining process.
[Fig. 14] Fig. 14 is a diagram illustrating a method for realizing the foregoing positioning.
[Fig. 15] Fig. 15 is a schematic plan view of the continuous sheet 5a of the back sheet in which different illustrations IL1 and IL2 are printed by a print pitch P5 in a direction in which the sheet 5a continues, the printing being performed so that the illustrations IL1 and IL2 are adjacent.

### [Description of Embodiments]

At least the following matters will be made clear by the description in the present specification and the accompanying drawings.

A method for manufacturing a sheet-like member associated with an absorbent article,
the manufacturing being performed by stacking and fixing a continuous first sheet and a continuous second sheet while aligning a direction in which the first sheet continues with a direction in which the second sheet continues,
the first sheet having a predetermined first pitch in the direction in which the first sheet continues,
   the first pitch being associated with a single piece of the absorbent article,
the second sheet having a display item at a second pitch in the direction in which the second sheet continues,
   the second pitch being smaller than the first pitch,
   the display item being formed before the second sheet becomes in a state of a sheet roll by being wound in the direction in which the second sheet continues,
the method including:
   feeding the second sheet by a feeding device from the sheet roll; and
   sending-off the second sheet by a sending-off device toward a merging position in a transportation path of the first sheet,
      the second sheet being fed and transported in a direction of transport,
      the direction of transport being the direction in which the second sheet continues,
   the feeding including:
      outputting a value with a first sensor; and
      performing by the feeding device based on the value a feeding operation of the second sheet from the sheet roll,
         the value depending on force that is applied to a center roll of three rolls by the second sheet wrapped around the three rolls,
         the three rolls being rotatably supported at a predetermined position upstream from the sending-off device in the direction of transport,
   the sending-off including
      changing by the sending-off device a stretching state of the second sheet in the direction of transport
      so that the display item of the second sheet being transported in the direction of transport is aligned at the first pitch, and
      so that the display item is located at a target position on the first sheet that is determined based on the first pitch and where the display item is to be placed.

With such a method for manufacturing a sheet-like member associated with an absorbent article, the value depending on force applied by the second sheet on the center roll of the three rolls is outputted from the first sensor. Based on the value, the feeding device performs feeding operation of the second sheet from the sheet roll. This force is force that increases/decreases in conjunction with the increase/decrease of the tension of the second sheet, and that can be an alternative index of the tension. Accordingly, as mentioned above, if the feeding operation is performed based on the value depending on the force, it is possible to reduce tension fluctuation of the second sheet which will be caused by change of the stretching state of the second sheet which is performed by the sending-off device. This makes it possible to send to the sending-off device the second sheet whose tension fluctuation is suppressed.

The foregoing three rolls are supported at the predetermined position, and these rolls rotate without substantial movement except for rotation. That is, basically, there is no translational movement. Accordingly, it is possible to substantially avoid the foregoing problem which will occur when at least one of these rolls performs translational move, that is, a problem that inertia-derived force of this roll due to translational movement causes another tension fluctuation of the second sheet. This makes it possible to send to the sending-off device the second sheet with its tension fluctuation being suppressed. This enables the sending-off device to change the stretching state of the second sheet with high accuracy. Consequently, in the sending-off step, the sending-off device can stack and fix the second sheet onto the first sheet with high accuracy so that the display item of the second sheet is aligned at the first pitch, and so that the display item is located at the target position on the first sheet that is determined based on the first pitch and where the display item is to be placed.

In such a method for manufacturing a sheet-like member associated with an absorbent article, it is preferable
that the feeding device includes a rotating shaft and a driving source coupled to the rotating shaft,
   the driving source driving and rotating the rotating shaft by applying rotational force to the rotating shaft, and
that the feeding operation is performed by driving and rotating the rotating shaft based on the value of the first sensor while the sheet roll being supported by the rotating shaft inserted to a through hole at a center of the sheet roll.

With such a method for manufacturing a sheet-like member associated with an absorbent article, the second sheet is fed from the sheet roll by driving and rotating the rotating shaft which is inserted to the through hole at the center of the sheet roll. This makes it possible to definitely avoid a trouble which will occur in a configuration in which the second sheet is fed from the sheet roll by driving and rotating the belt member which is in contact with the outer circum ferential surface of the sheet roll, that is, a trouble such as crease or looseness of a portion of the second sheet which is located on the outer circumferential surface of the sheet roll because of rubbing of the belt member on the outer circumferential surface, for example. This enables the feeding device to feed the second sheet toward the sending-off device with crease and looseness of the second sheet being substantially suppressed. This also contributes improvement of accuracy of positioning the display item to the target position, which is performed by the foregoing sending-off device.

In such a method for manufacturing a sheet-like member associated with an absorbent article, it is preferable that
the feeding operation is performed by the feeding device so that a tension-related value indicated by the value of the first sensor approaches a predetermined first target value.

With such a method for manufacturing a sheet-like member associated with an absorbent article, the feeding operation is performed by the feeding device so that the tension-related value indicated by the value of the foregoing first sensor approaches the predetermined first target value. Accordingly, the feeding operation by the feeding device can reliably reduce the tension fluctuation of the second sheet, which will be caused by changing the stretching state of the second sheet by the sending-off device.

In such a method for manufacturing a sheet-like member associated with an absorbent article, it is preferable that
the first target value is set so that a stretching ratio in the direction of transport of the second sheet at a position on the center roll is larger than a stretching ratio of the second sheet whose stretching state has been changed in the sending-off device.

With such a method for manufacturing a sheet-like member associated with an absorbent article, when feeding the second sheet, it is possible to make smaller the irregularity of its stretch/contraction ability which will exist in the sheet roll. This makes it possible for the sending-off device to change the stretching state of the second sheet with high accuracy. The detail is as follow. Generally speaking, when the second sheet becomes in a state of the sheet roll by being wound, the second sheet is being wound while being stretch by applying tension thereon in the direction in which the second sheet continues. At this stage, from the viewpoint of improving winding characteristics, tension value which is usually the value of tension is appropriately changed to a value suitable for an operation of winding. Consequently, the tension value is not constant. Accordingly, in the sheet roll, the stretching state of the second sheet is different depending on a position in the direction in which the second sheet continues. If the second sheet is left in the foregoing different stretching states for a certain time period, there is a possibility that the stretch/contraction ability of the second sheet becomes irregular during the time period. That is, some parts of the second sheet become more likely to stretch/contract and some other parts become less likely to stretch/contract, and those two types of part exist in the direction in which the second sheet continues.

If the foregoing sending-off device changes the stretching state of the second sheet whose stretch/contraction ability is irregular, the irregularity will affect the change, and consequently there is a possibility that the stretching state cannot be changed with high accuracy.

In this regard, in such a for manufacturing a sheet-like member, the foregoing first target value is set so that the stretching ratio of the second sheet at a position on the center roll for the feeding is larger than the stretching ratio of the second sheet whose stretching state is changed in the sending-off device. Accordingly, in the feeding, it is possible to stretch the second sheet so that a pitch of the display item which is aligned on the second sheet fed from the sheet roll is larger than the foregoing first pitch. Consequently, at a subsequent time when the sending-off device changes the stretching state of the second sheet, irregularity of the stretch/contraction ability of the second sheet is small at least until the alignment pitch of the display item becomes the foregoing first pitch. In other words, the irregularity is reduced. This makes is possible for the sending-off device to change the state in which the second sheet stretches in the direction of transport while reducing the influence by irregularity of the stretch/contraction ability, and therefore it is possible to change the state with high accuracy.

In such a method for manufacturing a sheet-like member associated with an absorbent article, it is preferable
that from the sending-off device toward the merging position, the second sheet is transported in a predetermined direction when viewed from above,
that at placement positions of the three rolls, the second sheet is transported along an intersecting direction when viewed from above, the intersecting direction intersecting the predetermined direction, and
that the direction of transport of the second sheet changes from the intersecting direction to the predetermined direction by wrapping the second sheet around a bar-shaped member,
   the bar-shaped member being arranged at a position in the direction of transport between the three rolls and the sending-off device.

With such a method for manufacturing a sheet-like member associated with an absorbent article, the bar-shaped member makes it possible to change the direction of transport of the second sheet from the intersecting direction to the predetermined direction when viewed from above. Accordingly, the foregoing three rolls can be placed at a position located away from the sending-off device in the intersecting direction (intersecting the predetermined direction when viewed from above; the predetermined direction connecting the sending-off device and the merging position). This can reduce the total length of a group of devices for this manufacturing method in the predetermined direction. This makes it possible to downsize the group of devices.

However, providing the bar-shaped member functioning to change the direction of transport makes it easier to cause such a trouble that, when tension fluctuation of the second sheet occurs at the position of the bar-shaped member, the second sheet is hooked to the bar-shaped member or sliding resistance between the second sheet and the bar-shaped member becomes excessively large. But, in this regard, such a manufacturing method makes it possible to substantially avoid occurrence of another tension fluctuation caused by inertia-derived force of the three rolls as mentioned above, and in other words the tension fluctuation of the second sheet is suppressed. Consequently, it is possible to effectively prevent the foregoing trouble from being induced.

In such a method for manufacturing a sheet-like member associated with an absorbent article, it is preferable
that a tension-adjusting device is provided at a position in the direction of transport between the three rolls and the sending-off device,
that the tension-adjusting device adjusts tension of the second sheet in the direction of transport,
that the tension-adjusting device includes three rolls and a driven roll,
   the three rolls being rotatably supported at a predetermined position
   the driven roll being located upstream from a center roll of the three rolls in the direction of transport,
   the driven roll being driven and rotated in order to come into contact with the second sheet to send-off the second sheet downstream in the direction of transport, and
that the tension of the second sheet is adjusted
   by outputting by a second sensor a value and
   by causing the driven roll to be driven and rotated based on the value,
      the value depending on force that is applied to the center roll by the second sheet wrapped around the three rolls of the tension-adjusting device.

With such a method for manufacturing a sheet-like member associated with an absorbent article, concerning tension fluctuation of the second sheet which will be caused by change of the stretching state of the second sheet which is performed by the sending-off device, the tension-adjusting device can reduce this tension fluctuation before the tension fluctuation is reduced by feeding operation of the feeding device. Accordingly, it is possible to further instantaneously reduce tension fluctuation. This enables the sending-off device to change the stretching state of the second sheet with higher accuracy.

Further, the foregoing three rolls are supported at the predetermined position, these rolls rotates without substantial movement except for rotation. That is, basically, there is no translational movement. Accordingly, it is possible to substantially avoid the foregoing problem which will occur when at least one of these rolls performs translational move, that is, a problem that inertia-derived force of this roll due to translational movement cause another tension fluctuation of the second sheet. This makes it possible to send to the sending-off device the second sheet with its tension fluctuation being suppressed. This enables the sending-off device to change the stretching state of the second sheet with high accuracy.

In such a method for manufacturing a sheet-like member associated with an absorbent article, it is preferable that
in a transportation path of the second sheet,
placement positions of the three rolls of the tension-adjusting device and a placement position of the driven roll are located closer to a sending-off position where the sending-off device sends-off the second sheet, than to a feeding position where the feeding device feeds the second sheet from the sheet roll.

With such a method for manufacturing a sheet-like member associated with an absorbent article, the foregoing tension-adjusting device can be placed close to the sending-off device. Accordingly, it is possible to further instantaneously reduce tension fluctuation of the second sheet which will be caused by change of the stretching state of the second sheet which is performed by the sending-off device. This enables the sending-off device to change the stretching state of the second sheet with much higher accuracy.

In such a method for manufacturing a sheet-like member associated with an absorbent article, it is preferable
that the driven roll is controlled to be driven and rotated so that a tension-related value indicated by the value of the second sensor approaches a predetermined second target value, and
that the second target value is set so that a stretching ratio in the direction of transport of the second sheet at a position on the center roll of the tension-adjusting device is smaller than both of
   a stretching ratio in the direction of transport of the second sheet at a position on the center roll for the feeding and
   a stretching ratio in the direction of transport of the second sheet whose stretching state has been changed in the sending-off device.

With such a method for manufacturing a sheet-like member associated with an absorbent article, the stretching ratio in the tension-adjusting device is smaller than the stretching ratio of the second sheet at the position on the center roll for the feeding, and is smaller than the stretching ratio of the second sheet whose stretching state has been changed in the sending-off device. Accordingly, troubles which will occur to the second sheet (e.g. shrinkage, longitudinal creases, or fold) can be prevented if in order to reduce the irregularity of the stretch/contraction ability, the second sheet is transported toward the sending-off device with keeping a great stretching ratio of the second sheet applied in the feeding device. This makes is possible for the sending-off device to change the stretching state of the second sheet with high accuracy. The "shrinkage" described herein is also referred to as "necking", and means a phenomenon that the widthwise size of the second sheet excessively shrinks (the width direction intersects the direction of transport). The term "longitudinal creases" means creases along a direction in which a sheet continues (the direction of transport). The term "fold" means that the widthwise ends of the second sheet fold inward in the width direction.

In such a method for manufacturing a sheet-like member associated with an absorbent article, it is preferable
that the sending-off device includes:
   a sending-off roll that is driven and rotated being in contact with the second sheet in order to send-off the second sheet downstream in the direction of transport;
   a detection sensor that detects a position of the display item and outputs a detection signal; and
   a controller that controls rotating operation of the sending-off roll based on the detection signal,
that when the detection signal indicates that the display item is displaced on the second sheet by a displacement amount upstream from the target position in the direction of transport,
   the controller increases a circumferential speed value of the sending-off roll based on the detection signal, and
   the tension-adjusting device changes a circumferential speed value of the driven roll,
      the changing being performed based on an amount of change in a direction in which the circumferential speed value increases based on the detection signal and based on the value outputted from the second sensor, and
that when the detection signal indicates that the display item is displaced on the second sheet by a displacement amount downstream from the target position in the direction of transport,
   the controller decreases the circumferential speed value of the sending-off roll, and
   the tension-adjusting device changes the circumferential speed value of the driven roll,
      the changing being performed based on an amount of change in a direction in which the circumferential speed value decreases based on the detection signal and based on the value outputted from the second sensor.

With such a method for manufacturing a sheet-like member associated with an absorbent article, the tension-adjusting device changes the circumferential speed value of the driven roll based on not only the value outputted from the second sensor of the tension-adjusting device but also the amount of change based on the detection signal which is outputted from the detection sensor as a result of detecting the position of the display item. Accordingly, the driven roll of the tension-adjusting device can rotate so that the tension fluctuation decreases, and this rotation can be made before the changing the stretching state by the sending-off device influences upstream as tension fluctuation of the second sheet and is detected by the second sensor of the tension-adjusting device. This makes it possible to suppress earlier tension fluctuation of the second sheet.

In such a method for manufacturing a sheet-like member associated with an absorbent article, it is preferable
that the feeding device is at least two feeding devices that feed the second sheet from the sheet roll,
that an accumulating device is provided at a position in the direction of transport between the feeding devices and the sending-off device,
   the accumulating device being capable of accumulating the second sheet in a form of loop,
when, in order to switch the feeding operation of the second sheet from one feeding device of the two feeding devices to another feeding device of the two feeding devices, the feeding operation being performed by the one feeding device is brought to stop,
   the accumulating device makes the loop smaller, to supply the second sheet to the sending-off device,
   a second sheet whose feeding operation in the one feeding device is stopped is joined to a second sheet of a sheet roll supported by the other feeding device,
   after the joining and after the other feeding device starts a feeding operation of the second sheet from the sheet roll, the accumulating device makes the loop larger, to accumulate the second sheet fed by the other feeding device,
that the accumulating device includes:
   a plurality of fixed rollers arranged at predetermined positions;
   a movable roller guided so as to reciprocate in a direction in which a size of the loop of the second sheet can change with respect to the fixed rollers;
   an actuator that reciprocates the movable roller in a direction of movement,
      the direction of movement being the direction in which the size of the loop of the second sheet can change, and
that the accumulating device forms the loop by wrapping the second sheet around the fixed roller and the movable roller,
   when the actuator moves the movable roller in a direction in which the movable roller approaches the fixed roller,
      the loop becomes smaller, to supply the accumulated second sheet to the sending-off device,
   when the actuator moves the movable roller in a direction in which the movable roller is away from the fixed roller,
      the loop becomes large to a predetermined length, to accumulate the second sheet,
   a period until a switching of the feeding operation includes a period during which the actuator holds the movable roller so that the movable roller stays at a predetermined position in the direction of movement.

With such a method for manufacturing a sheet-like member associated with an absorbent article, during the foregoing period for holding the movable roller, the movable roller is held by the actuator at the predetermined position in the direction of movement so as to stay at the predetermined position. This makes it possible to effectively suppress tension fluctuation of the second sheet which will be caused by inertia-derived force of the movable roller when the movable roller moves during the period.

In such a method for manufacturing a sheet-like member associated with an absorbent article, it is preferable that
when making the loop smaller,
the actuator is controlled so that a tension-related value indicated by the value of the first sensor approaches a predetermined third target value.

With such a method for manufacturing a sheet-like member associated with an absorbent article, it is possible to suppress tension fluctuation of the second sheet which will be caused when making the loop smaller. This makes it possible to send to the sending-off device the second sheet with its tension fluctuation being suppressed.

In such a method for manufacturing a sheet-like member associated with an absorbent article, it is preferable that
when, in order to make the loop larger, the actuator moves the movable roller to a position that corresponds to the predetermined length,
the feeding operation is performed by the other feeding device so that a tension-related value indicated by the value of the first sensor approaches a predetermined fourth target value.

With such a method for manufacturing a sheet-like member associated with an absorbent article, it is possible to suppress tension fluctuation of the second sheet which will be caused when making the loop larger. This makes it possible to send to the sending-off device the second sheet with its tension fluctuation being suppressed.

In such a method for manufacturing a sheet-like member associated with an absorbent article, it is preferable that
when making the loop smaller,
   the actuator moves the movable roller in the direction of movement toward a direction in which the loop becomes small, by applying to the movable roller a force in the direction,
when making the loop larger,
   the actuator moves the movable roller in the direction of movement toward a direction in which the loop becomes large, by applying to the movable roller a force in the direction.

With such a method for manufacturing a sheet-like member associated with an absorbent article, in either one of cases for making the loop smaller or larger, the actuator can receive and support the inertia-derived force of the movable roller. This makes it possible to avoid acting the inertia-derived force on the second sheet. Consequently, it is possible to effectively prevent the inertia-derived force from causing another tension fluctuation on the second sheet. Accordingly, the second sheet can be sent reliably to the sending-off device with its tension fluctuation being suppressed, as mentioned above.

In such a method for manufacturing a sheet-like member associated with an absorbent article, it is preferable that
that the feeding device is at least two feeding devices that feed the second sheet from the sheet roll, and
that when switching the feeding operation from one feeding device of the two feeding devices to another feeding device of the two feeding device,
   after detecting that a circumferential speed value of a sheet roll supported by the other feeding device increases to a same value as a feeding velocity of a second sheet in the one feeding device,
   a second sheet of the sheet roll supported by the other feeding device is joined to the second sheet fed by the one feeding device.

With such a method for manufacturing a sheet-like member associated with an absorbent article, after detecting that the circumferential speed value of the sheet roll of the other feeding device increases the feeding velocity of the second sheet of the one feeding device, the second sheet of the sheet roll supported by the other feeding device is joined to the second sheet fed by the one feeding device. Accordingly, the feeding operation can switch from the one feeding device to the other feeding device without stopping the feeding operation of the one feeding device. This makes it possible to omit the accumulating device of the second sheet which is necessary for stopping the feeding operation. Consequently, it is possible to simplify the configuration of the devices.

In such a method for manufacturing a sheet-like member associated with an absorbent article, it is preferable that
concerning the following two positions:
   a predetermined position on a display item of the second sheet fed by the one feeding device; and
   a position that is on a display item of the second sheet of the sheet roll supported by the other feeding device and that corresponds to the predetermined position,
when switching the feeding operation from the one feeding device to the other feeding device,
the second sheet of the sheet roll supported by the other feeding device is joined to the second sheet fed by the one feeding device while positioning so that a displacement amount in the direction of transport between the predetermined position and the position is within ±5% of the second pitch of the display item.

With such a method for manufacturing a sheet-like member associated with an absorbent article, the second sheet fed by the one feeding device and the second sheet of the sheet roll supported by the other feeding device are joined with such a positioning accuracy that the displacement amount of their display items in the direction of transport is within ±5% of the second pitch. Accordingly, continuity of the second sheet when switching feeding operation is substantially ensured about the state of alignment of the display item. This makes it unnecessary to excessively change stretching state, which the sending-off device will have to perform has to perform if discontinuity of the state of alignment of display item is large. This effectively contributes to change of the stretching state of the second sheet by the sending-off device with high accuracy.

Further,
A device for manufacturing a sheet-like member associated with an absorbent article,
the manufacturing being performed by stacking and fixing a continuous first sheet and a continuous second sheet while aligning a direction in which the first sheet continues with a direction in which the second sheet continues,
   the first sheet having a predetermined first pitch in the direction in which the first sheet continues,
      the first pitch being associated with a single piece of the absorbent article,
   the second sheet having a display item at a second pitch in the direction in which the second sheet continues,
      the second pitch being smaller than the first pitch,
      the display item being formed before the second sheet becomes in a state of a sheet roll by being wound in the direction in which the second sheet continues,
   the device including:
      a feeding device that feeds the second sheet from the sheet roll;
      a sending-off device that sends-off the second sheet toward a merging position in a transportation path of the first sheet,
         the second sheet being fed and transported in in a direction of transport,
         the direction of transport being the direction in which the second sheet continues;
      three rolls that are rotatably supported at a predetermined position in the direction of transport, the predetermined position being between the feeding device and the sending-off device; and
      a first sensor that outputs a value,
         the value depending on force that is applied to a center roll of three rolls by the second sheet wrapped around the three rolls,
      based on the value, the feeding device performing feeding operation of the second sheet from the sheet roll,
      while sending-off the second sheet, the sending-off device changing a stretching state of the second sheet in the direction of transport
         so that the display item of the second sheet being transported in the direction of transport is aligned at the first pitch, and
         so that the display item is located at a target position on the first sheet that is determined based on the first pitch and where the display item is to be placed.

With such an apparatus for manufacturing a sheet-like member associated with an absorbent article, it is possible to achieve the same effects as those of the foregoing method for manufacturing.

### = Present Embodiment =

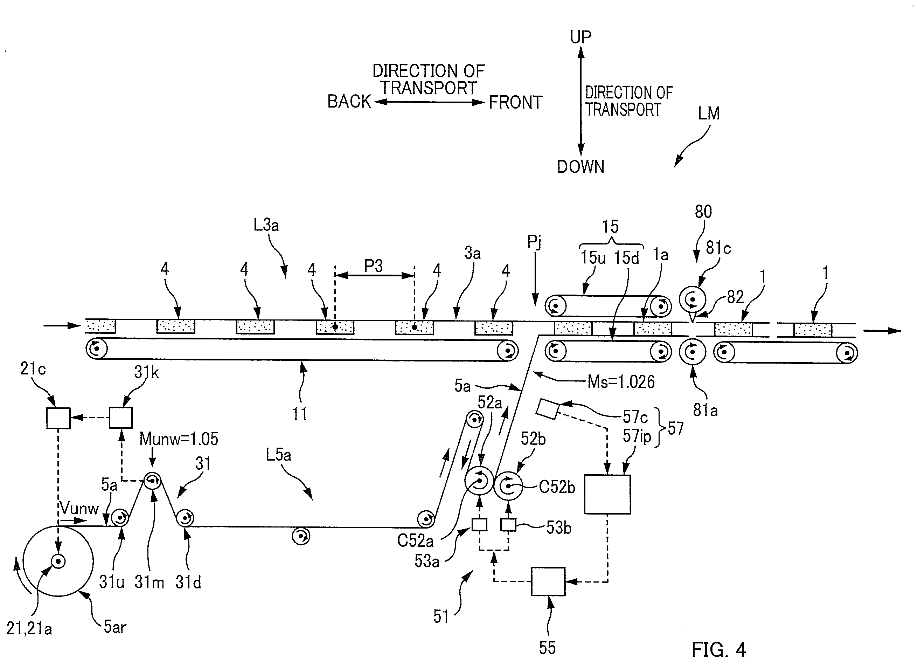

In a method for manufacturing a sheet-like member according to the present embodiment, manufactured is a continuous body 1a of the absorbent main body of a disposable diaper, which is as an example of the sheet-like member. After the continuous body 1a of the absorbent main body has been manufactured, the continuous body 1a is transported to a cutter device 80 (see Fig. 4) located downstream and is divided with the cutter device 80 by a product pitch P3 in the direction of transport, to produce the absorbent main body 1.

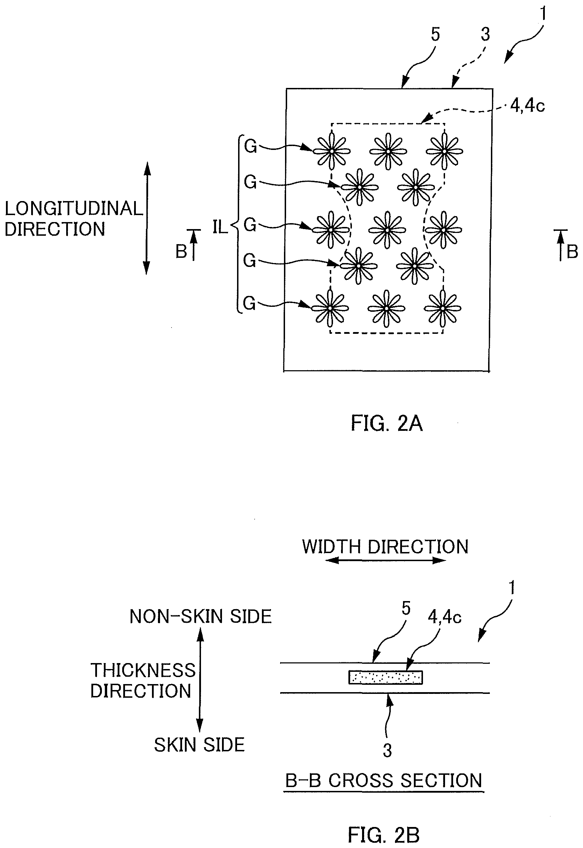

Fig. 2A is a schematic plan view of the absorbent main body 1 viewed from the non-skin side, and Fig. 2B is a cross-sectional view taken along line B-B of Fig. 2A. The absorbent main body 1 a sheet-like member having a longitudinal direction, the width direction and the thickness direction which are perpendicular to one another, and its shape is substantially rectangular when viewed from above. The absorbent main body 1 includes: an absorbent body 4 that absorbs liquid; a top sheet 3 that covers the absorbent body 4 from the skin side in the thickness direction; and a back sheet 5 that covers the absorbent body 4 from the non-skin side in the thickness direction. Onto the absorbent main body 1, an exterior sheet (not shown) or the like is placed and fixed and a diaper is substantially finished; the exterior sheet constitutes the exterior of a diaper and is in an hourglass shape when viewed from above.

The absorbent body 4 includes: an absorbent core 4c made of liquid absorbent material (e.g. pulp fiber and super absorbent polymer (SAP)) which is shaped into a certain shape such as substantially an hourglass shape when viewed from above; and a liquid-permeable core wrap sheet (not shown; e.g. tissue paper) which covers substantially the entire outer face of the absorbent core 4c. Note that the core wrap sheet may be omitted.

The top sheet 3 is a liquid-permeable sheet whose planar size is larger than that of the absorbent body 4. The material thereof is exemplified by air-through nonwoven fabric or the like, and air-through nonwoven fabric is used in this example. However, the present invention is not limited thereto as long as it is a liquid-permeable sheet.

The back sheet 5 is also a sheet whose planar size is larger than that of the absorbent body 4, but the back sheet 5 is liquid impermeable. The material thereof is exemplified by resin film made of polyethylene (PE), polypropylene (PP) or the like, and PE film is used in this example. However, the present invention is not limited thereto as long as it is a liquid-impermeable sheet having a certain stretch/contraction ability.

As shown in Fig. 2A, on the non-skin side surface of the back sheet 5, printed is an illustration IL of flower, which is an example of a display item. Specifically, the illustration IL includes a plurality of flower pictures G, G ... and the plurality of pictures G, G ... are combined to form a single illustration IL. Thus, the illustration IL is printed through substantially the entire surface of the back sheet 5. Printing the illustration IL through substantially the entire surface in such a manner relates to accuracy in positioning the illustration IL with respect to the continuous sheet 3a of the top sheet (to be described later); the positioning is performed in the manufacturing line LM of the continuous body 1a of the absorbent main body. The detail thereof will be described later. As a method for printing the illustration IL, there are gravure printing, flexography, ink-jet printing or the like.

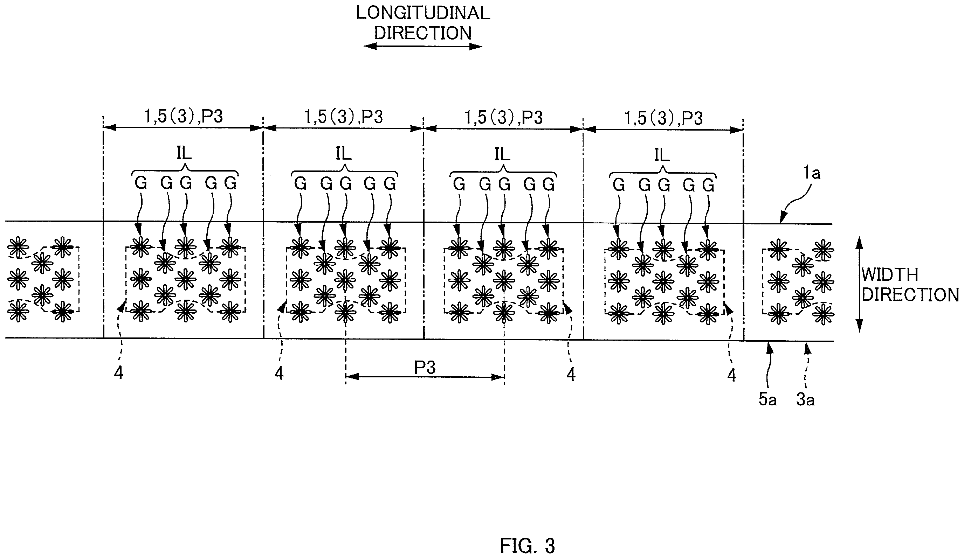

Fig. 3 is a schematic plan view of the continuous body 1a of the absorbent main body, which is to be the absorbent main body 1. The continuous body 1a of the absorbent main body is formed of a plurality of absorbent main bodies 1, 1 ... which continue in the longitudinal direction. Specifically, the continuous body 1a includes: the continuous sheet 3a of the top sheet (corresponding to the first sheet) which is formed of a plurality of the top sheets 3, 3 ... continuing in the longitudinal direction; the continuous sheet 5a of the back sheet (corresponding to the second sheet) which is formed of a plurality of the back sheets 5, 5, ... continuing in the longitudinal direction; and a plurality of the absorbent bodies 4, 4 ... which are placed between these continuous sheets 3a and 5a and which are aligned at the product pitch P3 (corresponding to the first pitch) in the longitudinal direction. These materials 3a, 5a and 4 are respectively bonded to their own adjacent material with hot-melt adhesive or the like.

The continuous body 1a of the absorbent main body is manufactured in the manufacturing line LM shown in the schematic side view of Fig. 4. The manufacturing line LM includes a processing system L3a for the continuous sheet 3a of the top sheet and a processing system L5a for the continuous sheet 5a of the back sheet. The processing system L5a for the continuous sheet 5a of the back sheet merges into the processing system L3a for the continuous sheet 3a of the top sheet at a merging position Pj which is located in the system L3a.

In this manufacturing line LM, the width direction of the manufacturing line LM (a direction penetrating the paper plane of Fig. 4) is defined as CD direction. In this example, CD direction is oriented in the horizontal direction. However, the present invention is not limited thereto. And, in this example, two directions perpendicular to CD direction are defined as a vertical, up-down direction and a horizontal, front-back direction. Accordingly, each of the directions in which the continuous sheets 3a and 5a of the top sheet and the back sheet are transported is oriented in a direction specified by the up-down direction and the front-back direction, depending on a position in the direction of transport. The width directions of the continuous sheets 3a and 5a of the top sheet and the back sheet are each parallel to CD direction. Directions perpendicular to CD direction and the direction of transport are defined as Z direction, and Z direction is parallel to the thickness directions of the continuous sheets 3a and 5a of the top sheet and the back sheet.

### <<< Processing System L3a for Continuous Sheet 3a of Top Sheet >>>

The processing system L3a for the continuous sheet 3a of the top sheet includes: a transport device 11 which transports the continuous sheet 3a of the top sheet along the direction of transport; and a bonding device provided according to the foregoing merging position Pj. At a time when the continuous sheet 3a is transported by the transport device 11, the absorbent bodies 4, 4 ... have already been fixed to the continuous sheet 3a at the product pitch P3 in the direction of transport. This transport operation is performed based on a synchronization signal. The synchronization signal is a signal consisting of a unit signal which corresponds to each transport operation by the product pitch P3; the unit signal is repeatedly outputted. In this example, the unit signal is a rotational angle signal having a rotational angle value of 0° to 360°. The synchronization signal is transmitted to an amplifier (not shown) of a servo motor (not shown), which serves as a driving source of the transport device 11. Accordingly, every time when a unit signal is outputted, the transport device 11 transports the continuous sheet 3a in the direction of transport by the product pitch P3.

The transport device 11 is exemplified by a belt conveyor or a transport roller. In this example, a belt conveyor is mainly used. The belt conveyor is exemplified by an ordinary belt conveyor having an endless belt which is driven and rotated, and which serves as a transport surface, and is exemplified by a suction belt conveyor having an endless belt whose outer circumferential surface has absorption function.

The synchronization signal can be generated, for example, by the foregoing cutter device 80, which divides the continuous body 1a of the absorbent main body by the product pitch P3 in the direction of transport to produce the absorbent main body 1. That is, the cutter device 80 includes a cutter roll 81c and an anvil roll 81a. The cutter roll 81c includes a cutter blade 82 on its outer circumferential surface and rotates along the direction of transport. The anvil roll 81a rotates the direction of transport while receiving the cutter blade 82 with its outer circumferential surface. On a CD direction end of the cutter roll 81c, a rotary encoder (not shown) is provided as an example of a rotation detector that detects the rotating operation of the cutter roll 81c. Every time when the cutter blade 82 rotates by the product pitch P3, the encoder outputs the foregoing unit signal in conjunction with the rotating operation of the cutter roll 81c.

The bonding device 15 includes, for example, a pair of upper and lower belt conveyors 15u and 15d. Between the pair of upper and lower belt conveyors 15u and 15d, formed is a transportation path for the continuous sheet 3a of the top sheet on which the absorbent bodies 4 have already been fixed. The foregoing merging position Pj is provided at an upstream position in the transportation path, and at the merging position Pj, the continuous sheet 5a of the back sheet is fed from the processing system L5a therefor to merge into the continuous sheet 3a of the top sheet. The continuous sheet 3a of the top sheet and the continuous sheet 5a of the back sheet are moving in the transportation path between the pair of belt conveyors 15u and 15d with being stacked in the thickness direction. During this movement, these sheets 3a and 5a are pressed in the thickness direction between the pair of belt conveyors 15u and 15d, and thus the sheets 3a and 5a being stacked are firmly bonded with hot-melt adhesive or the like.

Transport operation by the pair of belt conveyors 15u and 15d, which are included in the bonding device 15, is also performed based on the foregoing synchronization signal. That is, every time when a unit signal of the synchronization signal is outputted, the belt conveyors 15u and 15d transport, by the product pitch P3 in the direction of transport, the continuous sheet 3a of the top sheet and the continuous sheet 5a of the back sheet.

### <<< Processing System L5a for Continuous Sheet 5a of Back Sheet >>>

The processing system L5a for the continuous sheet 5a of the back sheet includes: a feeding device 21; a tension-measuring device 31; and a sending-off device 51. The feeding device 21 is for feeding from a sheet roll 5ar the continuous sheet 5a of the back sheet. The tension-measuring device 31 measures the tension of the fed continuous sheet 5a in the direction of transport while transporting the continuous sheet 5a in the direction of transport. When the continuous sheet 5a passes the tension-measuring device 31, the sending-off device 51 sends-off the continuous sheet 5a toward the merging position Pj in the processing system L3a, which is for the continuous sheet 3a of the top sheet. At the merging position Pj, the continuous sheet 5a and the continuous sheet 3a are stacked and fixed by the foregoing bonding device 15 as mentioned above, and thus the continuous body 1a of the absorbent main body is produced.

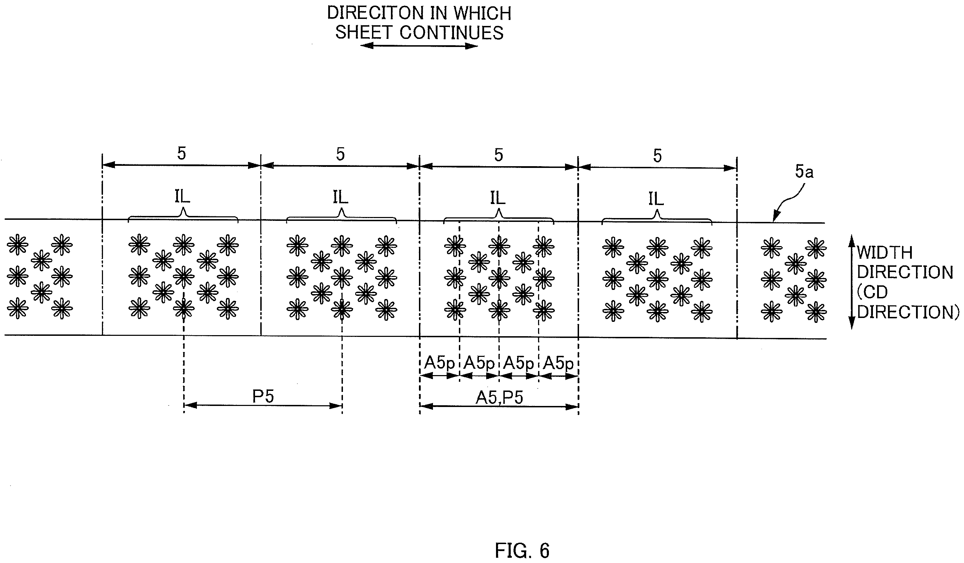

On the continuous sheet 5a of the back sheet, the foregoing illustrations IL, IL ... are printed at a certain print pitch P5 (corresponding to the second pitch) in the direction in which the sheet 5a continues (see Fig. 6), and the printing is performed in advance in another place or factory before the continuous sheet 5a are made in a form of sheet roll 5ar by being wound in the direction in which the sheet 5a continues. The print pitch P5 is smaller than the product pitch P3 which is employed on the continuous sheet 3a of the top sheet. Accordingly, in the bonding device 15 located at the foregoing merging position Pj, the continuous sheet 5a is stacked and fixed to the continuous sheet 3a basically with the continuous sheet 5a being stretched at the stretching ratio Ms (= P3/P5), which is obtained by dividing the product pitch P3 by the print pitch P5. Thereby the illustrations IL are arranged at appropriate positions on the continuous sheet 3a of the top sheet as shown in Fig. 3.

The stretching ratio described herein indicates, in the direction in which the continuous sheet 5a continues (the direction of transport), how many original unstretched lengths Ln (a length of the sheet on which no external force is applied) the total length Lt of the continuous sheet 5a in stretching state is. For example, the stretching ratio can be obtained as a dividing value (= Lt/Ln) dividing the total length Lt in the stretching state by the original unstretched length Ln.

In this example, if the print pitch P5 is "1", the product pitch P3 is "1.026". Accordingly, the stretching ratio Ms of the continuous sheet 5a in the bonding device 15 is 1.026. Since the stretching at the stretching ratio Ms is performed at a position between the sending-off device 31 and the bonding device 15, the stretching ratio Ms can be referred to as the stretching ratio Ms in the bonding device 15 as mentioned above, and also can be referred to as the stretching ratio Ms in the sending-off device 51.

The devices 21, 31 and 51 associated with the processing system L5a for the continuous sheet 5a of the back sheet will be described below.

### (1) Feeding Device 21

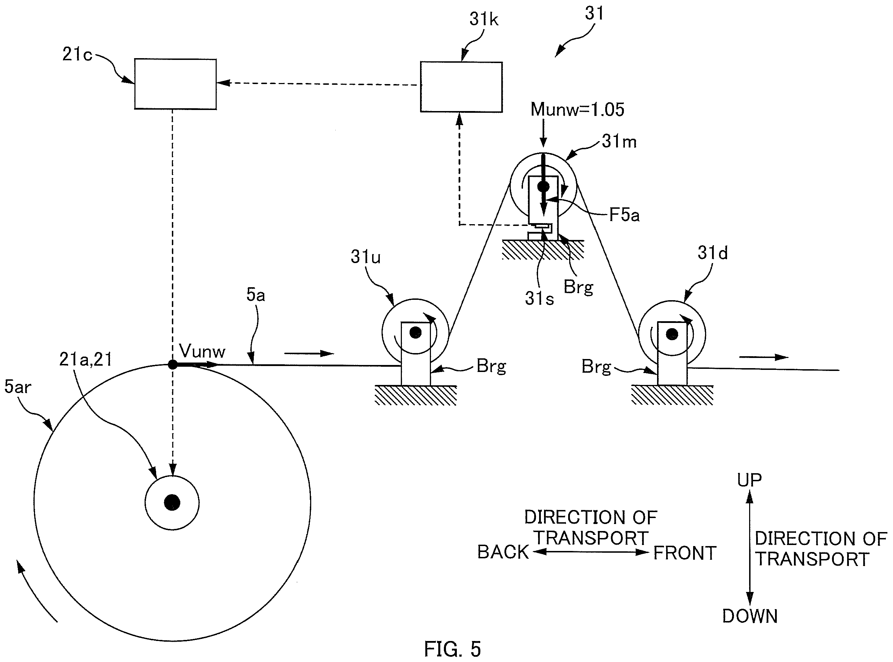

As shown in schematic magnified side view of Fig. 5, the feeding device 21 includes: a rotating shaft 21a; a driving source (not shown) coupled to the rotating shaft 21a; and a controller 21c. The driving source drives and rotates the rotating shaft 21a by applying rotational force to the rotating shaft 21a. The controller 21c controls the driving source based on a tension value outputted from the tension-measuring device 31. the continuous sheet 5a of the back sheet is fed from the sheet roll 5ar by driving and rotating the rotating shaft 21a based on the tension value while the sheet roll 5ar being supported by the rotating shaft 21a inserted to a through hole at the center of the sheet roll 5ar.

The rotating shaft 21a is a shaft member along CD direction, for example. The rotating shaft 21a is rotatably supported by an appropriate bearing member (not shown) provided in the manufacturing line LM.

The driving source is a servo motor, for example. In this example, as a method for connecting the servo motor and the rotating shaft 21a, a direct drive mechanism is employed. That is, the driving-rotating shaft (not shown) of the servo motor and the rotating shaft 21a are coaxially coupled with an appropriate shaft coupling (not shown). Thus, rotational force is directly applied to the rotating shaft 21a from the driving-rotating shaft which is driven and rotated by power supplied to the servo motor. This makes it possible to perform feeding operation with high response and without backlash. However, the present invention is not limited thereto. That is, when relatively low response is acceptable, rotational force of the driving-rotating shaft may be transmitted to the rotating shaft 21a through a plurality of things such as gears or timing belts, which are provided between the driving-rotating shaft of the servo motor and the rotating shaft 21a, for example.

The controller 21c is an appropriate computer such as a programmable logic controller (PLC) or a sequencer, and includes a processor and a memory. The processor reads and executes programs stored in the memory, and thereby the processor controls the feeding operation of the rotating shaft 21a. That is, the processor controls the feeding velocity Vunw (m/min) of the rotating shaft 21a so that the tension value (N) (corresponding to the "tension-related value") outputted from the tension-measuring device 31 matches a predetermined tension target value (corresponding to the "first target value").

More specifically, the foregoing control is performed, for example, by repeating the following process in a certain control period. First, the displacement amount of the tension value from the tension target value is obtained by subtracting the tension value from the tension target value. Next, the displacement amount is multiplied by a certain control gain to calculate an amount of control, and a new instruction value is obtained by adding the amount of control to the current instruction value of the feeding velocity Vunw (m/min). The rotating shaft 21a is rotated according to the new instruction value. Accordingly, if the tension value is larger than the tension target value, the feeding velocity Vunw (m/min) becomes larger than the current value by the absolute value of the amount of control, and this makes the tension value smaller to approach the tension target value. On the other hand, if the tension value is smaller than the tension target value, the feeding velocity Vunw (m/min) becomes smaller than the current value by the absolute value of amount of control, and this makes the tension value larger to approach the tension target value.

In cases where the feeding velocity Vunw is changed so that the tension value matches the tension target value in the foregoing manner, it is possible to reduce the fluctuation due to the change of the foregoing feeding velocity Vunw even if, when the sending-off device 51 (Fig. 4) located downstream in the direction of transport changes the stretching state of the continuous sheet 5a of the back sheet, the tension of the continuous sheet 5a fluctuates and the fluctuation influences upstream in the direction of transport. This enables the feeding device 21 to send to the sending-off device 51 the continuous sheet 5a whose tension fluctuation is suppressed. This enables the sending-off device 51 to change the stretching state of the continuous sheet 5a with high accuracy.

The foregoing feeding velocity Vunw (m/min) is, for example, the circumferential speed value (m/min) of the outer circumferential surface of the sheet roll 5ar. In a narrower sense, the feeding velocity Vunw (m/min) is the circumferential speed value (m/min) of the outer circumferential surface of the sheet roll 5ar at a position where the continuous sheet 5a of the back sheet separates away from the sheet roll 5ar (corresponding to the feeding position).

Meanwhile, the foregoing tension target value is set to a value according to a stretching ratio Munw which should be realized in the feeding device 21. That is, the tension target value is set to a load value obtained as follow: the load-displacement curve of the continuous sheet 5a of the back sheet is measured in advance, and a load value is obtained for a displacement corresponding to this stretching ratio Munw. Here, the value of the stretching ratio Munw is larger than the stretching ratio Ms of the continuous sheet 5a of the back sheet (Fig. 4) whose stretching state has been changed in the sending-off device 51. For example, in this example, the stretching ratio Munw is 1.05, and is larger than 1.026, the stretching ratio Ms of the foregoing sending-off device 31.

Thus, the feeding device 21 stretches in advance the continuous sheet 5a of the back sheet so that a pitch of the illustrations IL, IL ... which are aligned in the direction of transport on the continuous sheet 5a of the back sheet fed from the sheet roll 5ar is larger than the foregoing product pitch P3. Consequently, at a subsequent time when the sending-off device 51 changes the stretching state of the continuous sheet 5a of the back sheet, irregularity of the stretch/contraction ability of the continuous sheet 5a can be small at least until the alignment pitch of the illustrations IL, IL ... in the direction of transport becomes the product pitch P3. In other words, the irregularity can be reduced. This makes is possible for the sending-off device 51 to change the state in which the continuous sheet 5a stretches in the direction of transport while reducing the influence by irregularity of the stretch/contraction ability. This effectively contributes to change the stretching state with high accuracy.

In the example of Fig. 4, the stretching ratio Munw applied by the feeding device 21 is 1.05. The stretching ratio Mw is a ratio which is applied to the continuous sheet 5a of the back sheet (including the illustrations IL printed thereon) when winding the sheet 5a to be the sheet roll 5ar in another factory, and the stretching ratio Mw is smaller than the stretching ratio Munw, within a range from 1.005 to 1.03. That is, the stretching ratio Munw for stretching in the feeding device 21 is larger than the stretching ratio Mw for winding.

Accordingly, large stretching of the continuous sheet 5a of the back sheet in the feeding device 21 can reliably reduce irregularity of the stretch/contraction ability of the continuous sheet 5a which will be caused by leaving the continuous sheet 5a being wound in a form of sheet roll 5ar while being stretched at the stretching ratio Mw for winding. This makes is possible for the sending-off device 51 to change the stretching state of continuous sheet 5a with high accuracy when it sends-off the continuous sheet 5a towards the merging position Pj.

With reference to Fig. 2A, the illustration IL are printed on the non-skin side surface of a single sheet of the back sheet 5 as mentioned above, throughout substantially the entire surface. That is, in the continuous sheet 5a of the back sheet shown in Fig. 6, when a regionA5 corresponding to the print pitch P5 is divided into four equal-sized regions A5p in the direction in which the sheet 5a continues, the illustrations IL exist in all of the four regions A5p.

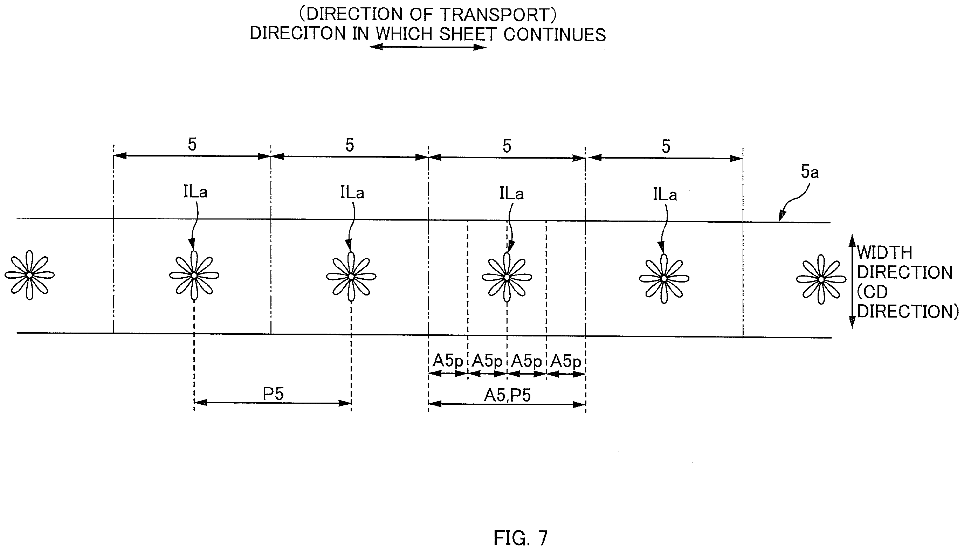

Accordingly, compared to a case where the illustration ILa exists in a narrow region as shown in Fig. 7, in the example of Fig. 6, it is possible to reliably reduce irregularity of the stretch/contraction ability of the continuous sheet 5a will be caused because of the illustrations IL. This effectively contributes to change of the stretching state of the continuous sheet 5a of the back sheet by the sending-off device 51 with high accuracy.

### (2) Tension-Measuring Device 31

As shown in Fig. 4, the tension-measuring device 31 includes three rolls 31u, 31m and 31d; these three rolls 31u, 31m and 31d are parallel to one another, and are rotatably supported at a predetermined position in the direction of transport downstream from the feeding device 21 and upstream from the sending-off device 21. The tension-measuring device 31 also includes a first sensor 31s (Fig. 5), and the first sensor 31s outputs in real time a value depending on force F5a (Fig. 5) which is applied by the continuous sheet 5a of the back sheet on the center roll 31m while the continuous sheet 5a of the back sheet being wrapped around three the rolls 31u, 31m and 31d in a form of mountain. Further, the tension-measuring device 31 includes a convertor 31k which convert to a tension value the foregoing value outputted by the first sensor 31s.

The three rolls 31u, 31m and 31d are each rotatably supported about a rotational axis along CD direction. That is, as shown in Fig. 5, on the CD direction ends of the rolls 31u, 31m and 31d, respective bearing members Brg are immovably provided at a predetermined position in the manufacturing line IM. Thus, the rolls 31u, 31m and 31d are supported by the bearing members Brg on their own both ends in such a manner to be capable of rotating and not to be capable of translational move. Concerning the center roll 31m of three the rolls 31u, 31m and 31d, its bearing members Brg each have a strain gauge 31s as an example of the foregoing first sensor 31s. The strain gauge 31s outputs in real time a value depending on force F5a applied on the roll 31m by the continuous sheet 5a of the back sheet. That is, the strain gauge 31s output in real time a value depending on the force F5a exerted on the roll 31m; the direction of the force F5a being in the radial direction of the roll 31m. The force F5a is force that increases/decreases in conjunction with the increase/decrease of the tension of the continuous sheet 5a of the back sheet. The force F5a is force which can be an alternative index of the tension, and therefore it is possible to calculate, from the foregoing value, the tension value which is the value of tension. The value outputted by the strain gauge 31s is inputted to the foregoing converter 31k, and in the convertor 31k, an appropriate coefficient or the like is multiplied to the value, to be converted to a tension value of the continuous sheet 5a of the back sheet. The tension value is inputted in real time to the foregoing controller 21c of the feeding device 21. In the controller 21c, the tension value is used for controlling the feeding velocity Vunw of the feeding device 21 (for example, calculating the displacement amount of this tension value from the tension target value).

The three rolls 31u, 31m and 31d are supported at the predetermined position as mentioned above, and therefore the three rolls rotates without substantial movement except for rotation. That is, basically, there is no translational movement. Accordingly, it is possible to substantially avoid the foregoing problem which will occur when at least one of these the rolls 31u, 31m and 31d performs translational move, that is, a problem that inertia-derived force of this roll due to translational movement causes another tension fluctuation of the continuous sheet 5a of the back sheet. This makes it possible to send to the sending-off device 51 the continuous sheet 5a with the fluctuation being suppressed. This enables the sending-off device 51 to change the stretching state of the continuous sheet 5a with high accuracy. Consequently, the sending-off device 51 can stack and fix the continuous sheet 5a onto the continuous sheet 3a with high accuracy so that illustrations IL, IL ... of the continuous sheet 5a are aligned at the product pitch P3 in the direction of transport, and so that the illustrations IL, IL ... are located at target positions on the continuous sheet 3a of the top sheet that are determined based on the product pitch P3 and where the illustrations IL are to be placed.

In this example, as shown in Fig. 5, the continuous sheet 5a of the back sheet is wrapped around the three rolls 31u, 31m and 31d in a form of mountain, which protrudes upward. However, the present invention is not limited thereto. That is, three the rolls 31u, 31m and 31d may be arranged so that the continuous sheet 5a is wrapped in a form of valley, which protrudes downward.

### (3) Sending-off Device 51

As shown in Fig. 4, the sending-off device 51 sends-off the continuous sheet 5a of the back sheet toward the merging position Pj in the processing system L3a for the continuous sheet 3a of the top sheet. When sending-off the continuous sheet 5a of the back sheet, the stretching state of the continuous sheet 5a in the direction of transport is appropriately changed. Thus, the illustrations IL of the continuous sheet 5a are aligned at the foregoing product pitch P3. In addition, the illustrations IL are located at target positions on the continuous sheet 3a which is determined based on the product pitch P3 and where the illustrations IL are to be placed.

The sending-off device 51 includes, for example, a pair of nip rolls 52a and 52b, a controller 55, and a detection sensor 57. The pair of nip rolls 52a and 52b are an example of the sending-off roll. The controller 55 controls the rotating operation of the pair of nip rolls 52a and 52b. The detection sensor 57 detects the positions of illustrations IL on the continuous sheet 5a of the back sheet.

The pair of nip rolls 52a and 52b are each rotatably supported about rotational axes C52a and C52b along CD direction. The nip rolls 52a and 52b obtains driving-and-rotational force from a servo motor (a driving source; not shown) and are rotating with sandwiching the continuous sheet 5a of the back sheet therebetween, and thereby sends-off the continuous sheet 5a toward the merging position Pj.

The controller 55 is an appropriate computer such as a PLC or a sequencer. The controller 55 controls the rotating operation of the nip rolls 52a and 52b based on detection signal of the illustration IL outputted from the detection sensor 57. This changes the stretching state of the continuous sheet 5a located between the bonding device 15 and the nip rolls 52a and 52b.

The detection sensor 57 includes, for example, an imaging device 57c and a the image processing device 57ip which processes image data transmitted from the imaging device 57c. The imaging device 57c includes, for example, a CCD camera, a processor and a memory. The camera images the continuous sheet 5a of the back sheet which is moving on its transportation path from the nip rolls 52a and 52b to the bonding device 15, for example.

Imaging operation is performed based on the foregoing synchronization signal. That is, the imaging device 57c is always receiving the synchronization signal, and when detecting that the rotational angle value of the synchronization signal matches a predetermined rotational angle value stored in advance in a memory of the imaging device 57c, the imaging device 57c performs imaging operation. The predetermined rotational angle value mentioned above is set to a value which makes the illustration IL be in an image indicated by image data, for example. Every time when the rotational angle value of the synchronization signal matches the predetermined rotational angle value, the imaging device 57c repeats imaging operation. Thus, in this example, imaging operation is performed for every illustration IL, to generate image data. Every time when another image data is generated, the other image data is transmitted to the image processing device 57ip.

The main body of the image processing device 57ip is an appropriate computer, and includes a processor and a memory. Every time when image data is transmitted from the imaging device 57c, the image processing device 57ip performs banalization based on the transmitted image data (the banalization is an example of image processing). By the banalization, pixels of an imaged part of the illustration IL, of the image of image data, are extracted, and the coordinates of the pixels are obtained. The detail is as follow.

First, the image of image data is composed of a plurality of pixels aligned in two dimensions: X direction and Y direction. The image is captured with CD direction being X direction and with the direction of transport being Y direction, for example. The image data has respective color information for each of the pixels. In this example, since the image data is of a grayscale image, the image data includes only brightness as color information for each pixel. The value of the brightness of each pixel indicating the illustration IL is lower than that of the brightness of each pixel indicating the continuous sheet 5a of the back sheet. Accordingly, a part of an image which the illustration IL is imaged can be extracted as a black image by banalization in which a pixel having a brightness equal to or greater than a certain threshold is assigned to a white image, and in which a pixel having a brightness less than the threshold is assigned to a black image. If the part which the illustration IL is imaged is extracted as a black image, the arithmetic mean of the coordinates of all pixels constituting the black image can be used as the coordinate representing the coordinates of the pixels of the part which the illustration IL is imaged.

In the foregoing memory of the image processing device 57ip, data of coordinates for comparison are stored in advance. The coordinates for comparison are coordinates where pixels of the illustration IL the image should be located if arranging the illustration IL to the target position defined on the continuous sheet 3a of the top sheet correctly performed at the merging position Pj. Y coordinate of the coordinates is the coordinate in the direction of transport.

Accordingly, a pixel of the part which the illustration IL is imaged is obtained by extracting in banalization; the value of Y coordinate of a pixel of the part which the illustration IL is imaged is subtracted from the value of Y coordinate form comparison. Thereby, it is possible for the image processing device 57ip to calculate the displacement amount of the illustration IL in the direction of transport. Every time when calculating the displacement amount, data of the calculated, displacement amount is transmitted as the detection signal to the controller 55.

Then, based on such a data, the controller 55 controls the rotating operation of the nip rolls 52a and 52b. For example, the amount of change ΔV is calculated by multiplying a predetermined gain by the displacement amount which is indicated by this data. And, a new instruction value is obtained by adding the amount of change ΔV to the current instruction value of the circumferential speed value (m/min), and based on the new instruction value, the amplifiers 53a and 53b of the servo motor of the nip rolls 52a and 52b are controlled. Thus, if the data indicates "an illustration IL is displaced upstream in the direction of transport", the circumferential speed value (m/min) increases from its current value by the absolute value of the amount of change ΔV, and this makes the amount of upstream shift of the illustration IL smaller. On the other hand, if the data indicates "an illustration IL is displaced downstream in the direction of transport", the circumferential speed value (m/min) decreases from its current value by the absolute value of the amount of change ΔV, and this makes the amount of downstream shift of the illustration IL smaller.

In this example, such a changing operation is performed every time when the foregoing data is transmitted to the controller 55. Accordingly, in the continuous sheet 5a of the back sheet, for all parts which is to be a single piece of diaper, the displacement amount is adjusted so as to be reduced. However, the present invention is not limited thereto. For example, the foregoing changing operation may be performed once for a plurality of transmitting operations.

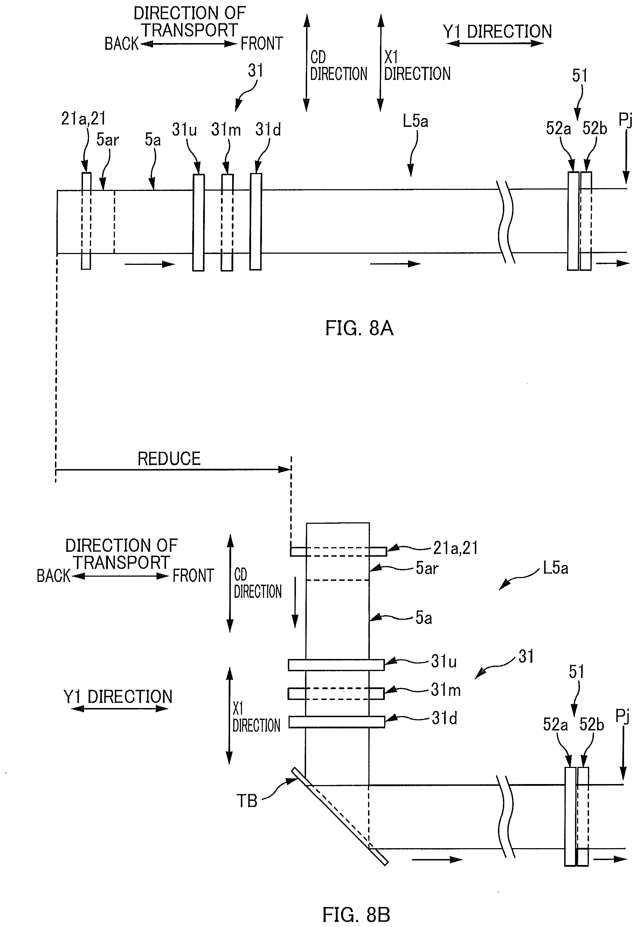

As shown in the schematic plan view of Fig. 8A, a direction along CD direction when the manufacturing line LM is viewed from above is defined as "X1 direction", and a direction perpendicular to CD direction is defined as "Y1 direction". In the foregoing example, throughout the entire length of the processing system L5a for the continuous sheet 5a of the back sheet, the direction of transport of the continuous sheet 5a is Y1 direction. Accordingly, at the placement position of the rotating shaft 21a of the feeding device 21 and at the placement positions of the three rolls 31u, 31m and 31d of the tension-measuring device 31, the direction of transport of the continuous sheet 5a is along Y1 direction. However, the present invention is not limited thereto. For example, the configuration shown in the schematic plan view of Fig. 8B is also acceptable.

That is, as shown in Fig. 8B, at the placement positions of the rotating shaft 21a and the rolls 31u, 31m and 31d, the direction of transport of the continuous sheet 5a may be set along an intersecting direction which intersects Y1 direction when viewed from above. More specifically, in the example of Fig. 8B, the direction of transport of the continuous sheet 5a at the foregoing placement positions is X1 direction. However, in this case, at a position between the placement positions the three rolls 31u, 31m and 31d of the tension-measuring device 31 and the placement position of the sending-off device 51, the direction of transport of the continuous sheet 5a has to change from X1 direction to Y1 direction. In this example, a turn bar TB (corresponding to the bar-shaped member) which is formed of a round bar having a diameter of 25.4 mm is placed at the foregoing position. Specifically, the turn bar TB is arranged in immovable and unrotatable manner with the longitudinal direction thereof being inclined by 45° with respect to both of X1 direction and Y1 direction. Accordingly, the continuous sheet 5a of the back sheet is wrapped around the turn bar TB, and as a result the direction of transport of the continuous sheet 5a changes from X1 direction to Y1 direction.

In the foregoing configuration including the turn bar TB as shown in Fig. 8B, the three rolls 31u, 31m and 31d of the tension-measuring device 31 and the rotating shaft 21a of the feeding device 21 can be placed at a position located away from the sending-off device 51 in X1 direction (a direction intersecting Y1 direction when viewed from above; Y1 direction connecting the sending-off device 51 and the merging position Pj) . This can reduce the total length of the manufacturing line IM in Y1 direction. This makes it possible to downsize the manufacturing line IM.

However, providing the turn bar TB makes it easier to cause such a trouble that, when tension fluctuation of the continuous sheet 5a occurs at the position of the turn bar TB, the continuous sheet 5a is hooked to the unrotating, turn bar TB or sliding resistance between the continuous sheet 5a and the turn bar TB becomes excessively large. But, in this example, occurrence of another tension fluctuation caused by inertia-derived force of the three rolls 31u, 31m and 31d is substantially avoided as mentioned above, and in other words the tension fluctuation of the continuous sheet 5a of the back sheet is suppressed. Consequently, it is possible to effectively prevent the foregoing trouble from being induced.

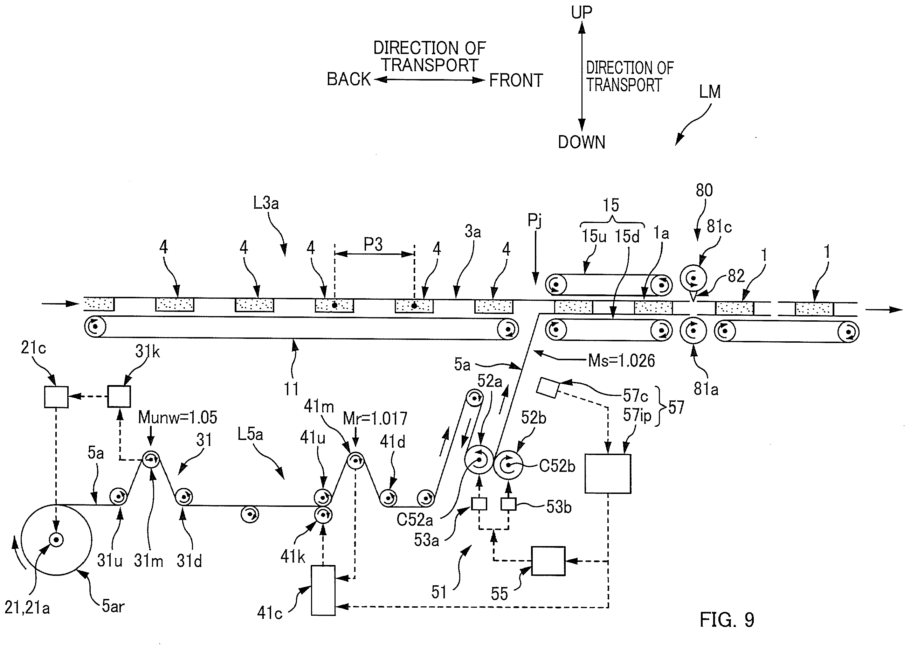

Fig. 9 is a schematic side view of a manufacturing line LM according to the first modified example of the present embodiment.

The main difference with the foregoing embodiment (Fig. 4) is that, as shown in Fig. 9, the first modified example includes a tension-adjusting device 41 which adjusts the tension of the continuous sheet 5a in the direction of transport, and that the tension-adjusting device 41 is located at a certain position between the nip rolls 52a and 52b (associated with the sending-off device 51) and the three rolls 31u, 31m and 31d (the tension-measuring device 31 in the processing system L5a for the continuous sheet 5a of the back sheet). The rest of the details is substantially the same as that of the foregoing embodiment. Hence, mainly this difference will be described below. The same components as those of the foregoing embodiment will be denoted by the same reference numerals, and the description thereof has been omitted.

The tension-adjusting device 41 includes three rolls 41u, 41m and 41d whose configurations are similar to the foregoing three rolls 31u, 31m and 31d of the tension-measuring device 31. Also, the tension-adjusting device 41 includes a second sensor (not shown), and the second sensor outputs in real time a value depending on force which is applied by the continuous sheet 5a of the back sheet on a center roll 41m of the three rolls 41u, 41m and 41d while the continuous sheet 5a of the back sheet being wrapped around the three rolls 41u, 41m and 41d in a form of mountain. The tension-adjusting device 41 also includes a driven roll 41k; the driven roll 41k is located upstream from the foregoing center roll 41m in the direction of transport, and is driven and rotated by a servo motor (not shown) in order to come into contact with the continuous sheet 5a to send-off it downstream in the direction of transport. Further, the tension-adjusting device 41 includes a controller 41c (e.g. a PLC or a sequencer) which controls the driven roll 41k based on a value outputted from the second sensor.

The three rolls 41u, 41m and 41d are each rotatably supported about a rotational axis along CD direction. That is, on the CD direction ends of the rolls 41u, 41m and 41d, respective bearing members (not shown) are immovably provided at a predetermined position in the manufacturing line IM. Thus, the rolls 41u, 41m and 41d are supported by the bearing members on their own both ends in such a manner to be capable of rotating and not to be capable of translational move. Concerning the center roll 41m of the three rolls 41u, 41m and 41d, its bearing members each have a strain gauge (not shown) as an example of the foregoing second sensor. The strain gauge outputs in real time a value depending on force applied on the roll 41m by the continuous sheet 5a of the back sheet. That is, the strain gauge outputs in real time a value depending on force exerted on the roll 41m; the direction of the force being in the radial direction of the roll 41m. The value outputted by the strain gauge is inputted to the foregoing controller 41c, and in the controller 41c an appropriate coefficient or the like is multiplied to the value, to be converted to a tension value (N) (corresponding to the "tension-related value") of the continuous sheet 5a of the back sheet. The controller 41c controls the circumferential speed value (m/min) of the driven roll 41k so that the tension value matches predetermined tension target value (corresponding to the "second target value").

More specifically, the foregoing control is performed, for example, by repeating the following process in a certain control period. First, the displacement amount of the tension value from the tension target value is obtained by subtracting the tension value from the tension target value. Next, the displacement amount is multiplied by a certain control gain to calculate an amount of control, and a new instruction value is obtained by adding the amount of control to the current instruction value of the circumferential speed value (m/min) . The driven roll 41k is rotated according to the new instruction value. Accordingly, if the tension value is larger than the tension target value, the circumferential speed value (m/min) becomes larger than the current value by the absolute value of the amount of control, and this makes the tension value smaller to approach the tension target value. On the other hand, if the tension value is smaller than the tension target value, the circumferential speed value (m/min) becomes smaller than the current value by the absolute value of the amount of control, and this makes the tension value larger to approach the tension target value.

In cases where the circumferential speed value of the driven roll 41k is changed so that the tension value matches the tension target value in the foregoing manner, it is possible to reduce the fluctuation due to the change of the circumferential speed value of the foregoing driven roll 41k even if, when the sending-off device 51 located downstream in the direction of transport changes the stretching state of the continuous sheet 5a of the back sheet, the tension of the continuous sheet 5a fluctuates and the fluctuation influences upstream in the direction of transport. This enables the tension-adjusting device 41 to send to the sending-off device 51 the continuous sheet 5a whose tension fluctuation is suppressed. This enables the sending-off device 51 to change the stretching state of the continuous sheet 5a with high accuracy.

The three rolls 41u, 41m and 41d are supported at the predetermined position as mentioned above, and therefore the three rolls rotates without substantial movement except for rotation. That is, basically, there is no translational movement. Accordingly, it is possible to substantially avoid the foregoing problem which will occur when at least one of these rolls 41u, 41m and 41d performs translational move, that is, a problem that inertia-derived force of this roll due to translational movement causes another tension fluctuation of the continuous sheet 5a of the back sheet. This makes it possible to send to the sending-off device 51 the continuous sheet 5a with the fluctuation being suppressed. This enables the sending-off device 51 to change the stretching state of the continuous sheet 5a with high accuracy.

In addition, if the tension-adjusting device 41 is provided, the tension-adjusting device 41 can reduce the foregoing tension fluctuation of the continuous sheet 5a due to the sending-off device 51 before the tension fluctuation is reduced by feeding operation of the feeding device 21. Accordingly, it is possible to further instantaneously reduce tension fluctuation. This enables the sending-off device 51 to change the stretching state of the continuous sheet 5a with higher accuracy.

From the viewpoint of instantaneousness, it is preferable that the tension-adjusting device 41 is placed close to the sending-off device 51. For this purpose, concerning the three rolls 41u, 41m and 41d of the tension-adjusting device 41 and the driven roll 41k, it is preferable that their placement positions in the transportation path of the continuous sheet 5a of the back sheet are located close to a sending-off position where the sending-off device 51 sends-off the continuous sheet 5a, than to a feeding position where the feeding device 21 feeds the continuous sheet 5a from the sheet roll 5ar.

In addition, from the viewpoint of instantaneousness, it is preferable that the controller 41c of the tension-adjusting device 41 controls the rotating operation of the driven roll 41k based on not only the tension value from the second sensor, but also the detection signal of illustration IL which is generated by the image processing device 57ip of the sending-off device 51 (that is, data indicating the displacement amount of the illustration IL in the direction of transport). For example, the foregoing detection signal is inputted from the image processing device 57ip to the controller 41c, and the controller 41c calculates amount of change ΔV by multiplying a predetermined gain by the displacement amount in the direction of transport which is indicated by the detection signal. And, a new instruction value is obtained by adding the amount of change ΔV and the amount of control associated with the tension value of the foregoing second sensor, to the current instruction value of the circumferential speed value of the driven roll 41k. It is preferable that the driven roll 41k rotates according to the new instruction value.

Accordingly, the driven roll 41k of the tension-adjusting device 41 can rotate so that the foregoing tension fluctuation becomes smaller, and this rotation can be made before changing the stretching state by the sending-off device 51 influences upstream as tension fluctuation of the continuous sheet 5a of the back sheet and is detected by the second sensor of the tension-adjusting device 41. This makes it possible to suppress earlier tension fluctuation of the continuous sheet 5a.

The tension-adjusting device 41 serves as a releasing device which releases the stretching state of the continuous sheet 5a which has been stretched by the feeding device 21. That is, the tension-adjusting device 41 releases the stretching state of the continuous sheet 5a so that the stretching ratio of the continuous sheet 5a is smaller than the stretching ratio Munw of the continuous sheet 5a when being stretched by the feeding device 21. In a case where the stretching state is released as mentioned above, it is possible effectively prevent troubles which will occur to the continuous sheet 5a (e.g. shrinkage, longitudinal creases, or fold) while being transported from the tension-adjusting device 41 to the sending-off device 51. In order to further improve effects of such prevention, it is preferable that the stretching ratio Mr of the continuous sheet 5a which has been released by the tension-adjusting device 41 is smaller than the stretching ratio Ms of the continuous sheet 5a whish has been changed by the sending-off device 51. In this example, for this purpose, the stretching ratio Mr is 1.017. The foregoing setting of the stretching ratio is made in the foregoing controller 41c by setting the foregoing tension target value to a value according to the stretching ratio Mr. A method for obtaining the tension target value is the same as that of the foregoing feeding device 21, and the description thereof has been omitted.

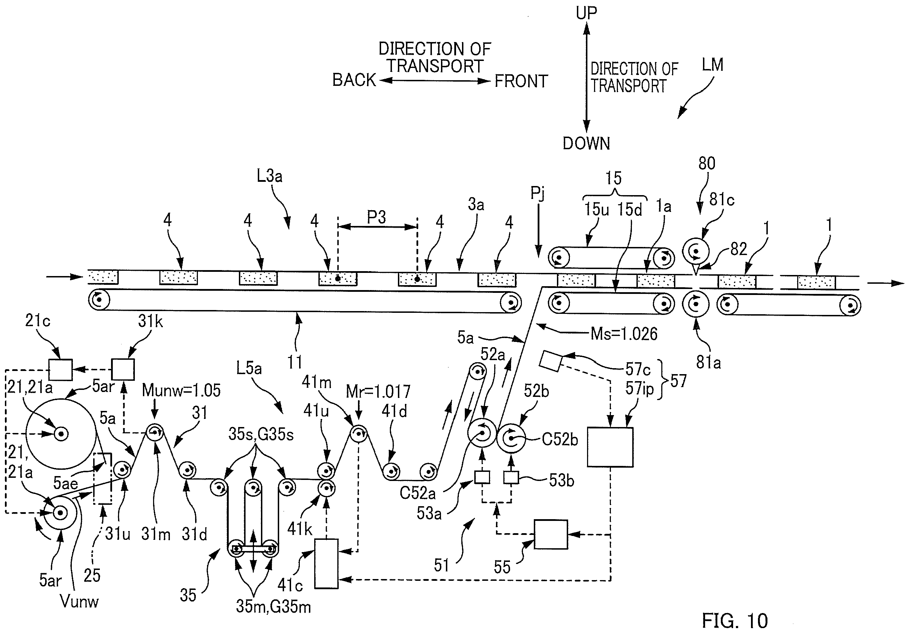

Fig. 10 is a schematic side view of a manufacturing line IM according to the second modified example of the present embodiment.

There are three main differences with the foregoing first modified example as follow: (1) two feeding devices 21 and 21 are provided; (2) a material-splicing device 25 is provided at a position in the direction of transport between the rotating shaft 21a of the feeding device 21 and three the rolls 31u, 31m and 31d of the tension-measuring device 31; and (3) an accumulating device 35 is provided between the material-splicing device 25 and the three rolls 41u, 41m and 41d of the tension-adjusting device 41, and is capable of accumulating the continuous sheet 5a of the back sheet in a form of loop. The rest of the details is substantially the same as that of the foregoing embodiment. Hence, mainly this difference will be described below. The same components as those of the foregoing embodiment will be denoted by the same reference numerals, and the description thereof has been omitted.

Each of the two feeding devices 21 includes the rotating shaft 21a. The configuration and operation of the rotating shaft 21a is the same as described in the present embodiment. For example, each of the rotating shafts 21a is driven and rotated by a servo motor (an example of a driving source), and the rotating shafts 21a are respectively inserted to through holes provided at the centers of the sheet rolls 5ar and 5ar. While supporting the sheet roll 5ar, the rotating shaft 21a is driven and rotated based on the foregoing tension value, which is basically derived from a value outputted by the first sensor 31s (Fig. 5). Thereby, the continuous sheet 5a of the back sheet is fed from the sheet roll 5ar.

But, the two feeding devices 21 and 21 are alternately switched and used. That is, during a time when the one feeding device 21 is feeding the continuous sheet 5a of the back sheet, the other feeding device 21 is substantially on standby. When the one feeding device 21 is about to run out of the continuous sheet 5a of the back sheet, the other feeding device 21 which is on standby replaces it and starts the feeding operation of the continuous sheet 5a of the back sheet.

The material-splicing device 25 a device which joins to the continuous sheet 5a being fed by the one feeding device 21 the continuous sheet 5a of a standby, sheet roll 5ar supported by the other feeding device 21. This makes it possible to continuously feed the continuous sheet 5a of the back sheet without interruption.

Such a material-splicing device 25 is a commonly-known component, and includes a clamp mechanism (not shown) and a cutter mechanism (not shown), for example. A joining process is performed as follow. First, the end part 5ae of the continuous sheet 5a of a standby sheet roll 5ar is placed by the other feeding device 21 onto one pinch member of the clamp mechanism, and double-faced tape is attached to the end part 5ae. Next, during a period when the one feeding device 21 stops feeding operation of the continuous sheet 5a of the back sheet, the one pinch member moves toward another pinch member facing the continuous sheet 5a, and a pair of these pinch members pinch the continuous sheet 5a, double-faced tape and end part 5ae. Thereby, the continuous sheet 5a which stops to be fed by the one feeding device 21 is joined to the continuous sheet 5a of the sheet roll 5ar which is on standby in the other feeding device 21. During a period when being pinched in such a manner, the cutter mechanism cuts the continuous sheet 5a in the one feeding device 21 at a position upstream in the direction of transport from a part joined with double-faced tape. Thereafter, the pair of pinch members release their pinching, and the sheet roll 5ar which feeds the continuous sheet 5a of the back sheet is switched from the sheet roll 5ar of the one feeding device 21 to that of the other feeding device 21.

The accumulating device 35 is a device which accumulates the continuous sheet 5a of the back sheet fed by the feeding device 21 in such a manner to be capable of being supplied downstream in the direction of transport. The accumulating device 35 includes a fixed-roller group G35s, a movable-roller group G35m, an actuator (not shown) and a controller (e.g. PLC; not shown). The fixed-roller group G35s is composed of a plurality of fixed rollers 35s, 35s... arranged at predetermined positions. The movable-roller group G35m is composed of a plurality of movable rollers 35m, 35m which are guided so as to reciprocate in the up-down direction; the size of the loop of the continuous sheet 5a can change in the up-down direction. The actuator reciprocates the movable-roller group G35m in the up-down direction, and the controller controls the actuator.

The continuous sheet 5a of the back sheet is wrapped alternately around the fixed rollers 35s belonging to the fixed-roller group G35s and the movable rollers 35m belonging to the movable-roller group G35m. Thus, the loop of the continuous sheet 5a of the back sheet is formed. Accordingly, if the movable-roller group G35m moves in a direction away from the fixed-roller group G35s (downward in the example of Fig. 10), the loop becomes large, to accumulate the continuous sheet 5a of the back sheet. On the other hand, in the up-down direction, if the movable-roller group G35m moves in a direction in which the movable-roller group G35m approaches the fixed-roller group G35s (upward in the example of Fig. 10), the loop becomes small, to supply the accumulated continuous sheet 5a to the sending-off device 51.

As mentioned above, when switching the feeding operation from the one feeding device 21 to the other feeding device 21, the feeding velocity Vunw of the continuous sheet 5a by the one feeding device 21 gradually decreases, to bring the feeding operation to stop. In this case, the accumulating device 35 makes the loop smaller, to supply the continuous sheet 5a of the back sheet to the sending-off device 51. Accordingly, without being affected by stop of the feeding operation, the sending-off device 51 can continuously perform send-offing operation while changing the stretching state. This supplying operation by the accumulating device 35 continues until the joining process of the continuous sheet 5a by the material-splicing device 25 has completed. Then, when the joining process is completed and the feeding operation by the other feeding device 21 is ready to start, the accumulating device 35 makes the loop larger to accumulate the continuous sheet 5a fed by the other feeding device 21. When the loop is in the predetermined length, the actuator holds the movable-roller group G35m until switching to the next feeding operation so that the movable-roller group G35m stays at a predetermined position in the up-down direction which corresponds to the foregoing predetermined length. This makes it possible to suppress tension fluctuation of the continuous sheet 5a which will be caused by inertia-derived force of each movable roller 35m when the movable-roller group G35m moves.

As such an actuator, used is one capable of conducting position control, and a linear servo motor is used in this example. The linear servo motor includes: a base member; a slider member which is guided so as to reciprocate in the up-down direction relative to the base member; and a linear encoder which measures the actual position of the slider member in the up-down direction. On the slider member, the movable rollers 35m of the movable-roller group G35m are rotatably supported being connected to respective bearing members (not shown) . As mentioned above, the linear servo motor moves the slider member in the up-down direction based on the foregoing position control. More specifically, the linear servo motor moves the slider member so as to make small a difference between the actual position of the slider member outputted from the linear encoder and an instructed position in the up-down direction which is indicated with a control signal transmitted by the controller. Accordingly, the controller transmits to the linear servo motor a control signal containing the instructed position (the predetermined position in this example), and this makes it possible to realize keeping the movable-roller group G35m at the predetermined position as mentioned above.

An operation of supplying the continuous sheet 5a by the accumulating device 35 when switching feeding operation is performed as follow. First, the tension value outputted by the tension-measuring device 31 is always inputted to the foregoing controller. When the feeding operation of the one feeding device 21 is brought to stop for the foregoing switching, the one feeding device 21 stops feeding the continuous sheet 5a. Nevertheless, the continuous sheet 5a is sent toward the sending-off device 51 located downstream, and this will make larger the tension value outputted from the tension-measuring device 31. But, at this stage, the controller controls the foregoing linear servo motor so that the tension value matches the predetermined tension target value (corresponding to the third target value). That is, the controller calculates to obtain an instructed position and transmits to the linear servo motor control signal containing the instructed position, and thereby the controller controls the motor so that the tension value matches the tension target value. Accordingly, the linear servo motor moves the slider member so as to return to the tension target value the tension value which is getting larger. That is, the movable-roller group G35m moves upward so that the loop becomes small, and consequently the continuous sheet 5a is supplied, without stopping, from the accumulating device 35 to the sending-off device 51 located downstream.

After the joining process is completed, the accumulating operation of the continuous sheet 5a by the accumulating device 35 is performed as follow. First, since this timing is immediately after switching the feeding operation, the feeding operation by the other feeding device 21 has already started at this timing. The feeding operation is performed based on the tension value outputted from the tension-measuring device 31 in the same manner as described above. That is, the continuous sheet 5a is fed from the other feeding device 21 so that the tension value matches a tension target value (corresponding to the fourth target value). Accordingly, when the controller controls positioning the linear servo motor to move downward the movable-roller group G35m to the foregoing predetermined position, the feeding device 21 feeds the continuous sheet 5a whose amount is the total of an amount necessary to be sent downstream toward the sending-off device 51 and an extra amount corresponding to the foregoing downward movement. Consequently, while keeping a continuous sheet 5a to be sent to the sending-off device 51, the loop is made larger and the continuous sheet 5a is accumulated to the accumulating device 35.

It is preferable that, when making the loop smaller, the linear servo motor moves upward the movable-roller group G35m by applying to the movable-roller group G35m a force which directs upward in the up-down direction, that is, toward a direction in which the loop becomes small. Also, it is preferable that, when making the loop larger, the linear servo motor moves downward the movable-roller group G35m by applying to the movable-roller group G35m a force which directs downward in the up-down direction, that is, toward a direction in which the loop becomes large.

In this case, the linear servo motor can receive and support inertia-derived force of the movable-roller group G35m. This makes it possible to avoid acting the inertia-derived force on the continuous sheet 5a. Consequently, it is possible to effectively prevent the force from causing another tension fluctuation on the continuous sheet 5a. Accordingly, the continuous sheet 5a can be sent reliably to the sending-off device 51 with its tension fluctuation being suppressed, as mentioned above.

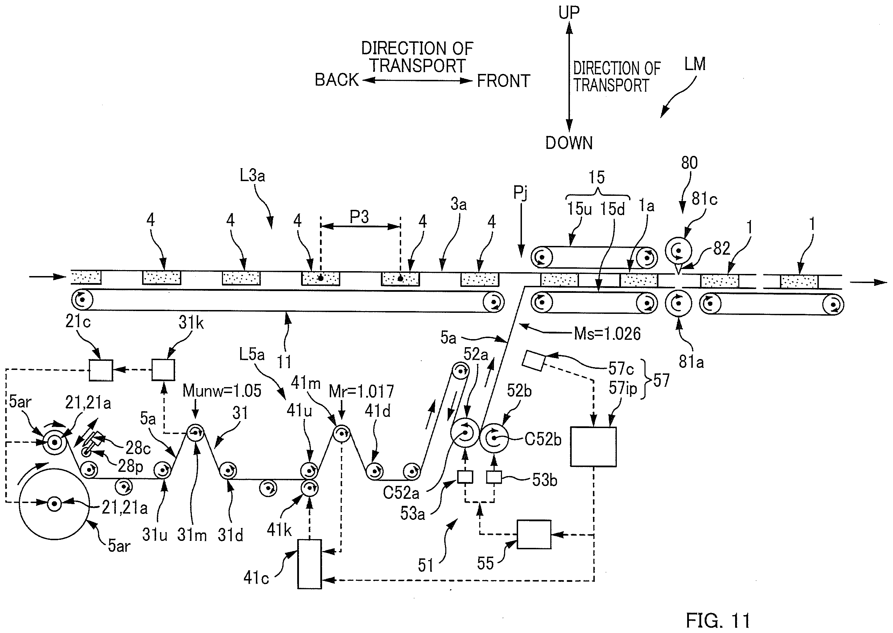

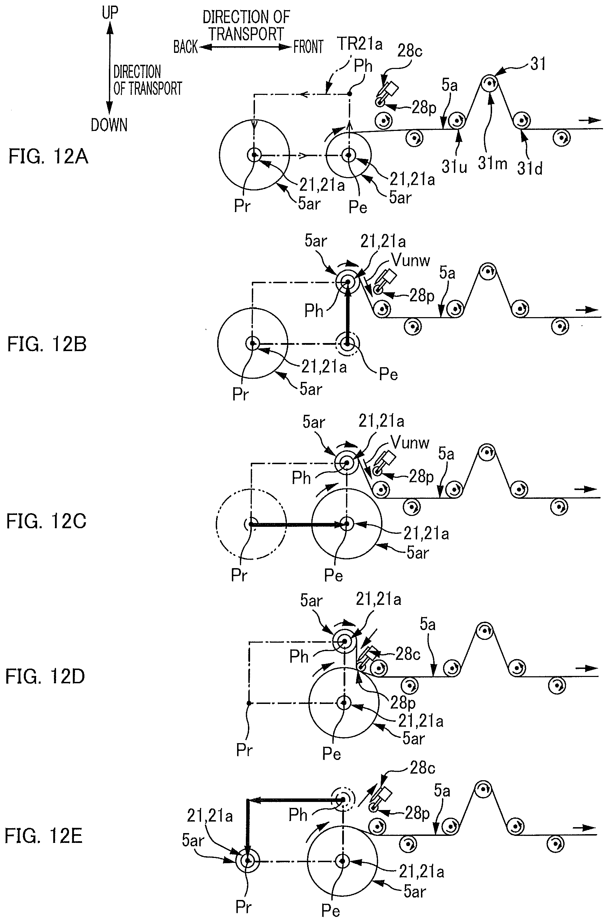

Fig. 11 is a schematic side view of a manufacturing line IM according to the third modified example of the present embodiment. Figs. 12A to 12E are schematic side views for illustrating the joining process performed in the third modified example.

In the foregoing second modified example, when feeding operation of the continuous sheet 5a fed by the one feeding device 21 stops, the material-splicing device 25 shown in Fig. 10 joins the continuous sheet 5a fed by the one feeding device 21 to the continuous sheet 5a of the sheet roll 5ar which is on standby in the other feeding device 21. Whereas, in the third modified example of Fig. 11, without stopping the feeding operation of the continuous sheet 5a by the one feeding device 21, the continuous sheet 5a which is being fed by the one feeding device 21 is joined to the continuous sheet 5a of the sheet roll 5ar which is on standby in the other feeding device 21. Accordingly, in the third modified example, the accumulating device 35 necessary for stopping feeding operation is omitted. The rest of the details is substantially the same as that of the second modified example. The same components as those of the second modified example will be denoted by the same reference numerals, and the description thereof has been omitted.

As shown in Fig. 12A, the rotating shafts 21a and 21a of two feeding devices 21 and 21 each move along rectangular track path TR21a, for example. In the track path TR21a, the following positions are determined: a feeding operation position Pe where the rotating shaft 21a performs most of feeding operation; a temporary-escape position Ph where the rotating shaft 21a which is soon going to finish feeding operation temporarily escapes; and a standby position Pr where the rotating shaft 21a which has finished feeding operation stays until next feeding operation. Switching feeding operation from one rotating shaft 21a to the other rotating shaft 21a is performed for example as follows.

As shown in Fig. 12A, the one rotating shaft 21a is located at the feeding operation position Pe, and the rotating shaft 21a is driven and rotated by a servo motor (not shown), to feed the continuous sheet 5a of the back sheet from the sheet roll 5ar. When the feeding operation is going to finish soon, the rotating shaft 21a moves with keeping its feeding operation, from the feeding operation position Pe to the temporary-escape position Ph located upward, as shown in Fig. 12B. After the rotating shaft 21a has moved to the temporary-escape position Ph, the rotating shaft 21a continues feeding operation without interruption.

Simultaneously with or after the foregoing movement, the other rotating shaft 21a moves from the standby position Pr to the feeding operation position Pe as shown in Fig. 12C. At this stage, to the other rotating shaft 21a, a new sheet roll 5ar has already attached when it was at the standby position Pr. When the other rotating shaft 21a has moved to the feeding operation position Pe, the other rotating shaft 21a is driven and rotated by a servo motor (not shown), to accelerate the circumferential speed value (m/min) of the outer circumferential surface of the sheet roll 5ar in the other rotating shaft 21a. When a suitable sensor (e.g. a rotary encoder) detects that the circumferential speed value becomes the same value as the feeding velocity Vunw (m/min) of the continuous sheet 5a of the one rotating shaft 21a at the temporary-escape position Ph, the circumferential speed value keeps to a value identical to the feeding velocity Vunw.

Then, as shown in Fig. 12D, the continuous sheet 5a fed by the one rotating shaft 21a placed at the temporary-escape position Ph is pressed by a roll-shaped, pressing member 28p which is capable of reciprocating. Thus, the continuous sheet 5a approaches to the outer circumferential surface of the sheet roll 5ar in the other rotating shaft 21a placed at the feeding operation position Pe, and is pressed against the outer circumferential surface. Then, the continuous sheet 5a is joined to the outer circumferential surface with an adhesion member (e.g. double-faced tape; not shown) which is provided in advance on the outer circumferential surface at a position where the foregoing continuous sheet 5a is pressed. The continuous sheet 5a is cut between the foregoing adhesion member and the one rotating shaft 21a, and thus the feeding operation is switched from the old sheet roll 5ar in the one rotating shaft 21a to the new sheet roll 5ar in the other rotating shaft 21a.

Then, as shown in Fig. 12E, the one rotating shaft 21a moves to the standby position Pr. During a period when the one rotating shaft 21a is on standby at the standby position Pr, the old sheet roll 5ar is removed from the one rotating shaft 21a and a new sheet roll 5ar is attached to the same.

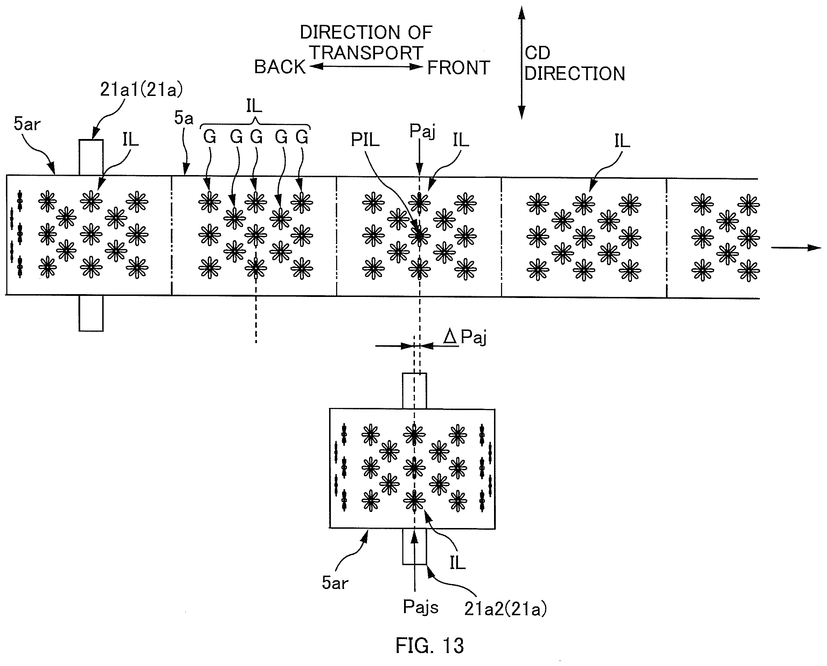

In Fig. 13, the following two positions are shown: a predetermined position Paj on an illustration IL of the continuous sheet 5a fed by the one rotating shaft 21a1 (in the example of Fig. 13, the center position PIL on the illustration IL); and a position Pajs which is on an illustration IL of the continuous sheet 5a of the sheet roll 5ar supported by the other rotating shaft 21a2 and which corresponds to the foregoing predetermined position Paj. Concerning these two positions, it is preferable that, when switching the feeding operation from the one rotating shaft 21a1 (21a) to the other rotating shaft 21a2 (21a), positioning is performed so that the position Paj and the position Pajs match in the direction of transport as shown in the schematic plan view of Fig. 13. Specifically, it is preferable that the continuous sheet 5a of the sheet roll 5ar supported by the other rotating shaft 21a2 is joined to the continuous sheet 5a fed by the one rotating shaft 21a1 while positioning so that the displacement amount ΔPaj in the direction of transport between the position Paj and the position Pajs is within ±5% (preferably ±1%) of the print pitch P5 of the illustrations IL (Fig. 6).

In this case, continuity of the continuous sheet 5a when switching feeding operation is substantially ensured about the state of alignment of the illustration IL. This makes it unnecessary to excessively change stretching state, which the sending-off device 51 will have to perform if discontinuity of the state of alignment of illustration IL is large. This effectively contributes to change of the stretching state of the continuous sheet 5a by the sending-off device 51 with high accuracy.

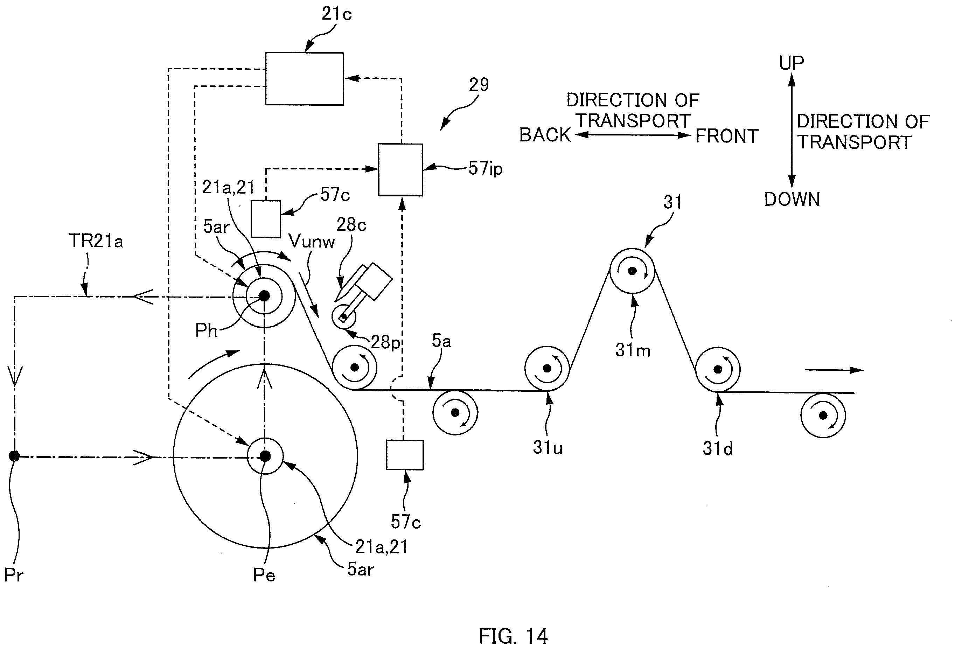

Such a positioning can be realized by using a sensor 29 (Fig. 14) similar to the detection sensor 57 (Fig. 4) used in the foregoing sending-off device 51. Fig. 14 is a schematic side view for describing such a configuration. As shown in Fig. 14, the sensor 29 includes: two imaging devices 57c and 57c; and the image processing device 57ip which processes image data transmitted from these two imaging devices 57c and 57c. One of two imaging devices 57c and 57c is provided being associated with the temporary-escape position Ph, and detects the illustration IL of the continuous sheet 5a which is being fed at the position Ph from the old sheet roll 5ar at the feeding velocity Vunw. The other imaging device 57c is provided being associated with the feeding operation position Pe. Concerning a new sheet roll 5ar which is accelerated to the circumferential speed value same as the feeding velocity Vunw to rotate at the circumferential speed value, the other imaging device 57c detects the illustration IL of the continuous sheet 5a which is being fed at the feeding operation position Pe from the new sheet roll 5ar. Both of the imaging devices 57c and 57c performs imaging operation based on the foregoing synchronization signal. For example, every time when the rotational angle value the synchronization signal matches the predetermined rotational angle value, these two imaging devices 57c and 57c performs imaging operation.

Every time when image data is transmitted from the two imaging devices 57c and 57c, the image processing device 57ip performs a process such as banalization to each of the transmitted image data. Thereby, pixels of an imaged part of the illustration IL, of the image of each image data, are extracted, and the coordinates of the pixels are obtained. The value of Y coordinate of the illustration IL on the continuous sheet 5a of the new sheet roll 5ar is subtracted from the value of Y coordinate of the illustration IL on the continuous sheet 5a of the old sheet roll 5ar. Thus, subtraction value is obtained, and is transmitted to the controller 21c which controls the rotating operation of the rotating shafts 21a and 21a.

By experiments or the like, found are in advance the range of the subtraction value which realizes the foregoing positioning state, that is, the range of the subtraction value which realizes a positioning state in which the displacement amount ΔPaj in the direction of transport is within ±5% of the print pitch P5 of the illustration IL. The range is stored in advance in a memory of the foregoing the controller 21c. Accordingly, if the subtraction value transmitted from the image processing device 57ip is not within the foregoing range, a processor of the controller 21c obtains an acceleration value (m/min²) by multiplying the subtraction value by a certain control gain. By the acceleration value, the rotating shaft 21a located at the feeding operation position Pe is accelerated. After this acceleration, by a deceleration operation which is substantially inverted operation of the acceleration operation, the circumferential speed value of the sheet roll 5ar returns to the circumferential speed value before the acceleration. Thus, while keeping the circumferential speed value of the new sheet roll 5ar to a value identical to the foregoing feeding velocity Vunw, the positions of the illustrations IL on the continuous sheet 5a of the new sheet roll 5ar moves downstream in the direction of transport relative to the positions of the illustrations IL on the continuous sheet 5a of the old sheet roll 5ar. This makes it possible to approach the positions of the new sheet roll 5ar to the positions of the old sheet roll 5ar. By repeating the foregoing operation for every imaging operation, the foregoing positioning is realized.

Meanwhile, in the foregoing second modified example (Fig. 10), the foregoing positioning may be made; that is, positioning may be made so that the displacement amount ΔPaj in the direction of transport is within ±5% of the print pitch P5 of the illustrations IL. But, in the second modified example, when the continuous sheet 5a of an old sheet roll 5ar is joined to the continuous sheet 5a of a new sheet roll 5ar, the material-splicing device 25 stops not only the feeding operation from the new sheet roll but also feeding operation from the old sheet roll 5ar. Accordingly, in this case, the foregoing positioning can be made by an operator by hand. The positioning by hand can be considered to be understandable without further explanation, and therefore the description thereof has been omitted.

### = Other Embodiments =

While the embodiment according to the invention is described above, the above-mentioned embodiment is provided for facilitating the understanding of the invention, and is not to be interpreted as limiting the invention. As a matter of course, the invention can be altered and improved without departing from the gist thereof and the invention includes equivalent thereof. For example, the invention can be altered as described below.

In the foregoing embodiment and the like, the first sheet is exemplified by the continuous sheet 3a of the top sheet, and the second sheet is exemplified by the continuous sheet 5a of the back sheet. However, the present invention is not limited thereto. For example, the following configuration is also acceptable: the first sheet is the continuous sheet of the exterior sheet which is an exterior of a diaper, and the second sheet is the continuous sheet of an illustration sheet which is bonded to the skin side of the exterior sheet and whose illustration can be visually recognized through the exterior sheet. Also, the continuous sheet of a leakage-proof sheet on which the illustrations IL are printed may be employed.

In the foregoing embodiment and the like, the display item is exemplified by the illustration IL, and the illustration IL is formed of a combination of a plurality of pictures G, G .... However, the present invention is not limited thereto. For example, the illustration IL may be formed of only one picture G as shown in Fig. 7. Or, the illustration IL may be formed of pictures which are neither an animal, a plant (e.g. flower) nor a mascot; for example, the illustration IL may be formed of letters or geometric pattern. Furthermore, a method for forming the display item is not limited to printing. For example, the picture of the display item may be formed as follow: the continuous sheet 5a of the back sheet (the second sheet) is pressed in the thickness direction, to form a plurality of dotted depressions or a plurality of linear depressions.

In the foregoing embodiment and the like, the unit signal of the synchronization signal is a signal indicated by a rotational angle value of 0° to 360°. However, the present invention is not limited thereto. For example, the unit signal of the synchronization signal may be a digital value (e.g. 0 to 8191). In the foregoing description, the synchronization signal is generated by the rotary encoder provided in the cutter device 80 shown in Fig. 4. However, the present invention is not limited thereto. For example, the synchronization signal may be generated by an appropriate controller (e.g. PLC; not shown) . That is, the unit signal of the synchronization signal may be generated repeatedly as follow: the controller includes a processor and a memory, the memory stores in advance a program to generate the synchronization signal, the processor reads the foregoing program from memory and executes it. Or, the synchronization signal may be generated by an appropriate electric circuit, not by such a processor which reads the foregoing program.

In the foregoing embodiment and the like, the detection sensor 57 associated with the sending-off device 51 is exemplified by the device including the imaging device 57c and the image processing device 57ip. However, the present invention is not limited thereto. That is, a sensor which is being capable of detecting the illustrations IL and obtaining their displacement amount in the direction of transport may be employed. For example, a sensor including a phototube may be used. That is, the rotating operation of the nip rolls 52a and 52b of the sending-off device 51 may be controlled based on a difference between: a time at which the illustration IL passing the position of the phototube is detected and the detection signal thereof is outputted by the phototube; and a time at which the synchronization signal having the predetermined rotational angle value stored in advance in a memory is outputted.

In the foregoing embodiment and the like, the absorbent article is exemplified by the disposable diaper. However, the present invention is not limited thereto as long as an article is one which absorbs excrement of a wearer. For example, a sanitary napkin, a urine absorbing pad or the like may be employed as an absorbent article.

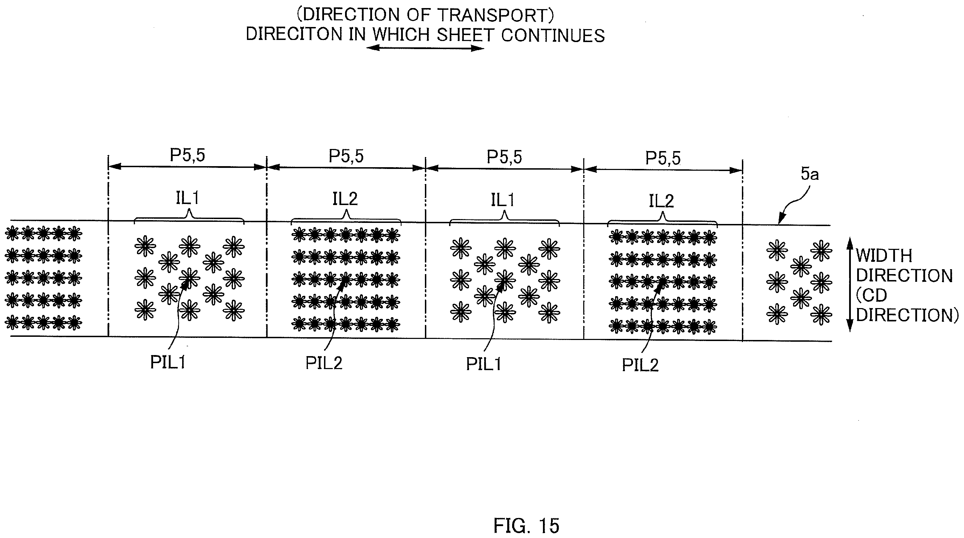

In the foregoing embodiment and the like, the display item is exemplified by illustration IL, and the illustrations IL and IL which are adjacent by the print pitch P5 in the direction in which the continuous sheet 5a of the back sheet continues are identical as shown in Fig. 6. However, the present invention is not limited thereto. That is, as shown in Fig. 15, illustrations IL1 and IL2 whose structures are different may be employed. But, in this case, the illustrations IL1 and IL2 whose structures are different are printed so that the center positions PILl and PIL2 of the illustrations IL1 and IL2 in the direction of transport are located at an identical position within each region corresponding to the print pitch P5. This makes it possible for the foregoing detection sensor 57 to calculate a correct displacement amount of the illustrations IL1 and IL2 in the direction of transport even if these illustrations IL1 and IL2 have different structures.

In the foregoing embodiment and the like, as shown in Fig. 4, the feeding device 21 is exemplified by the configuration including: the rotating shaft 21a which is inserted to a through hole at the center of the sheet roll 5ar, and which supports the sheet roll 5ar in a rotatable manner; and the driving source which is coupled to the rotating shaft 21a and which drives and rotates the rotating shaft 21a by applying rotational force to the rotating shaft 21a. However, the present invention is not limited thereto. For example, as shown in Fig. 1, the following configuration is acceptable: a belt member 21' is provided being in contact with the outer circumferential surface of the sheet roll 5ar and is driven and rotated, and thereby the continuous sheet 5a of the back sheet is fed from the sheet roll 5ar. But, in this configuration, the belt member 21' rubs the outer circumferential surface of the sheet roll 5ar, and this will cause troubles such as crease or looseness of a portion of the continuous sheet 5a which is located on the outer circumferential surface. Accordingly, there is a possibility that the continuous sheet 5a is fed toward the sending-off device 51 with crease and looseness being produced thereon. In order to prevent such a trouble, it is preferable that the feeding device 21 according to the foregoing embodiment is used.

In the foregoing embodiment and the like, "a tension-related value indicated by the value of the first sensor" is exemplified by the "tension value". However, the present invention is not limited thereto. That is, a value other than the tension value may be employed as long as the value increases/decreases in conjunction with the increase/decrease of the tension value. For example, the following values are also acceptable: a value obtained by multiplying the tension value by a certain constant (including fractions); a value obtained by adding a certain second constant (including negative values); and a combination of these values. In this case, it goes without saying that tension target values corresponding to "a first target value, a third target value and a fourth target value" according to the claims are determined according to the foregoing values.

In the foregoing first to third modified examples, "a tension-related value indicated by the value of the second sensor" is exemplified by the "tension value". However, the present invention is not limited thereto. That is, a value other than the tension value may be employed as long as the value increases/decreases in conjunction with the increase/decrease of the tension value. For example, the following values are also acceptable: a value obtained by multiplying the tension value by a certain constant (including fractions); a value obtained by adding a certain second constant (including negative values); and a combination of these values. In this case, it goes without saying that a tension target value corresponding to "a second target value" according to the claims are determined according to the foregoing value.

In the foregoing second modified example, as shown in Fig. 10, the accumulating device 35 is arranged at a position downstream from the tension-measuring device 31 in the direction of transport. However, the present invention is not limited thereto. For example, the accumulating device 35 may be arranged at a position upstream from the tension-measuring device 31 in the direction of transport. More specifically, the accumulating device 35 may be arranged between the material-splicing device 25 and the tension-measuring device 31.

In the foregoing embodiment and the like, the first sensor 31s is exemplified by the strain gauge. However, the present invention is not limited thereto. For example, a load cell may be employed.

In the foregoing first to third modified examples, the second sensor is exemplified by the strain gauge. However, the present invention is not limited thereto. For example, a load cell may be employed.

In the foregoing second modified example, supplying operation by the accumulating device 35 at the time of stopping the feeding operation is performed as follow: the controller controls the linear servo motor to move upward the movable-roller group G35m based on the tension value outputted from the tension-measuring device 31. However, the present invention is not limited thereto. For example, assuming that it is possible to estimate in the dimension of time the amount of the continuous sheet 5a per unit time (m) which is required to be sent-off downstream to the sending-off device 51 when the feeding operation is brought to stop. In this case, it is possible to calculate a pattern of moving-up operation (a graph of the relation between moving-up velocity (m/min) and time (sec.)), which is for moving upward the movable-roller group G35m without loosing and largely pulling the continuous sheet 5a when the feeding operation is brought to stop. Accordingly, the foregoing supplying operation may be realized as follow: the pattern of operation is stored in advance in a memory of the controller in a form of program; and based on the program, a processor of the controller performs position controlling by the linear servo motor.

In the foregoing second modified example, at a relatively early timing after the supplying operation of the continuous sheet 5a, the accumulating device 35 accumulates the continuous sheet 5a until the length of the accumulated sheet 5a reaches to the predetermined length. Thereafter, until next switching of feeding operation, the movable-roller group G35m is held in position controlling by the linear servo motor so that the movable-roller group G35m stays at the predetermined position in the up-down direction which corresponds to the foregoing predetermined length. However, the present invention is not limited thereto. For example, after performing supplying operation, the accumulating device 35 may perform the accumulating operation immediately before next switching of feeding operation. In this case, until the timing when the accumulating operation starts, the accumulating device 35 keeps the loop of the continuous sheet 5a in a state in which the loop is small immediately after the supplying operation. It is preferable that the movable-roller group G35m is held in position controlling by the linear servo motor so that the movable-roller group G35m stays at the predetermined position in the up-down direction which corresponds to the loop. This makes it possible to effectively suppress tension fluctuation of the continuous sheet 5a which will be caused by inertia-derived force of each movable roller 35m when the movable rollers 35m move during a period approximately from the end of the supplying operation to the start of the accumulating operation.

### [Reference Signs List]

1 absorbent main body, 1a continuous body of absorbent main body (sheet-like member), 3 top sheet, 3a continuous sheet of top sheet (first sheet), 4 absorbent body, 4c absorbent core, 5 back sheet, 5a continuous sheet of back sheet (second sheet), 5ae end part, 5ar sheet roll, 11 transport device, 15 bonding device, 15u belt conveyor, 15d belt conveyor, 21 feeding device, 21a rotating shaft, 21a1 rotating shaft, 21a2 rotating shaft, 23 belt conveyor, 21c controller, 25 material-splicing device, 28p pressing member, 29 sensor, 31 tension-measuring device, 31k convertor, 31u roll, 31m roll, 31d roll, 31s strain gauge (first sensor), 35 accumulating device, 35m movable roller, 35s fixed roller, 41 tension-adjusting device, 41c controller, 41k driven roll, 41u roll, 41m roll, 41d roll, 51 sending-off device, 52a nip roll, 52b nip roll, 53a amplifier, 53b amplifier, 55 controller, 57 detection sensor, 57c camera (imaging device), 57ip the image processing device, 80 cutter device, 81a anvil roll, 81c cutter roll, 82 cutter blade, LM manufacturing line, L3a processing system of continuous sheet of top sheet, L5a processing system of continuous sheet of back sheet, Brg bearing member, TB turn bar (bar-shaped member), G35m movable-roller group, G35s fixed-roller group, TR21a track path, C52a rotational axis, C52b rotational axis, IL illustration (display item), IL1 illustration (display item), IL2 illustration (display item), ILa illustration (display item), A5 region, A5p region, G picture, F5a force, Pj merging position, Pe feeding operation position, Ph temporary-escape position, Pr standby position, PIL center position, PILl center position, PIL2 center position, Paj predetermined position, Pajs position corresponding to predetermined position,

## Claims

1. A method for manufacturing a sheet-like member associated with an absorbent article,
the manufacturing being performed by stacking and fixing a continuous first sheet and a continuous second sheet while aligning a direction in which the first sheet continues with a direction in which the second sheet continues,
the first sheet having a predetermined first pitch in the direction in which the first sheet continues,
the first pitch being associated with a single piece of the absorbent article,
the second sheet having a display item at a second pitch in the direction in which the second sheet continues,
the second pitch being smaller than the first pitch,
the display item being formed before the second sheet becomes in a state of a sheet roll by being wound in the direction in which the second sheet continues,
the method comprising:
feeding the second sheet by a feeding device from the sheet roll; and
sending-off the second sheet by a sending-off device toward a merging position in a transportation path of the first sheet,
the second sheet being fed and transported in a direction of transport,
the direction of transport being the direction in which the second sheet continues,
the feeding including:
outputting a value with a first sensor; and
performing by the feeding device based on the value a feeding operation of the second sheet from the sheet roll,
the value depending on force that is applied to a center roll of three rolls by the second sheet wrapped around the three rolls,
the three rolls being rotatably supported at a predetermined position upstream from the sending-off device in the direction of transport,
the sending-off including
changing by the sending-off device a stretching state of the second sheet in the direction of transport
so that the display item of the second sheet being transported in the direction of transport is aligned at the first pitch, and
so that the display item is located at a target position on the first sheet that is determined based on the first pitch and where the display item is to be placed.

2. A method for manufacturing a sheet-like member associated with an absorbent article according to claim 1, wherein
the feeding device includes a rotating shaft and a driving source coupled to the rotating shaft,
the driving source driving and rotating the rotating shaft by applying rotational force to the rotating shaft, and
the feeding operation is performed by driving and rotating the rotating shaft based on the value of the first sensor while the sheet roll being supported by the rotating shaft inserted to a through hole at a center of the sheet roll.

3. A method for manufacturing a sheet-like member associated with an absorbent article according to claim 1 or 2, wherein
the feeding operation is performed by the feeding device so that a tension-related value indicated by the value of the first sensor approaches a predetermined first target value.

4. A method for manufacturing a sheet-like member associated with an absorbent article according to claim 3, wherein
the first target value is set so that a stretching ratio in the direction of transport of the second sheet at a position on the center roll is larger than a stretching ratio of the second sheet whose stretching state has been changed in the sending-off device.

5. A method for manufacturing a sheet-like member associated with an absorbent article according to any one of claims 1 to 4, wherein
from the sending-off device toward the merging position, the second sheet is transported in a predetermined direction when viewed from above,
at placement positions of the three rolls, the second sheet is transported along an intersecting direction when viewed from above, the intersecting direction intersecting the predetermined direction, and
the direction of transport of the second sheet changes from the intersecting direction to the predetermined direction by wrapping the second sheet around a bar-shaped member,
the bar-shaped member being arranged at a position in the direction of transport between the three rolls and the sending-off device.

6. A method for manufacturing a sheet-like member associated with an absorbent article according to any one of claims 1 to 5, wherein
a tension-adjusting device is provided at a position in the direction of transport between the three rolls and the sending-off device,
the tension-adjusting device adjusts tension of the second sheet in the direction of transport,
the tension-adjusting device includes three rolls and a driven roll,
the three rolls being rotatably supported at a predetermined position
the driven roll being located upstream from a center roll of the three rolls in the direction of transport,
the driven roll being driven and rotated in order to come into contact with the second sheet to send-off the second sheet downstream in the direction of transport, and
the tension of the second sheet is adjusted
by outputting by a second sensor a value and
by causing the driven roll to be driven and rotated based on the value,
the value depending on force that is applied to the center roll by the second sheet wrapped around the three rolls of the tension-adjusting device.

7. A method for manufacturing a sheet-like member associated with an absorbent article according to claim 6, wherein
in a transportation path of the second sheet,
placement positions of the three rolls of the tension-adjusting device and a placement position of the driven roll are located closer to a sending-off position where the sending-off device sends-off the second sheet, than to a feeding position where the feeding device feeds the second sheet from the sheet roll.

8. A method for manufacturing a sheet-like member associated with an absorbent article according to claim 6 or 7, wherein
the driven roll is controlled to be driven and rotated so that a tension-related value indicated by the value of the second sensor approaches a predetermined second target value, and
the second target value is set so that a stretching ratio in the direction of transport of the second sheet at a position on the center roll of the tension-adjusting device is smaller than both of
a stretching ratio in the direction of transport of the second sheet at a position on the center roll for the feeding and
a stretching ratio in the direction of transport of the second sheet whose stretching state has been changed in the sending-off device.

9. A method for manufacturing a sheet-like member associated with an absorbent article according to any one of claims 6 to 8, wherein
the sending-off device includes:
a sending-off roll that is driven and rotated being in contact with the second sheet in order to send-off the second sheet downstream in the direction of transport;
a detection sensor that detects a position of the display item and outputs a detection signal; and
a controller that controls rotating operation of the sending-off roll based on the detection signal,
when the detection signal indicates that the display item is displaced on the second sheet by a displacement amount upstream from the target position in the direction of transport,
the controller increases a circumferential speed value of the sending-off roll based on the detection signal, and
the tension-adjusting device changes a circumferential speed value of the driven roll,
the changing being performed based on an amount of change in a direction in which the circumferential speed value increases based on the detection signal and based on the value outputted from the second sensor, and
when the detection signal indicates that the display item is displaced on the second sheet by a displacement amount downstream from the target position in the direction of transport,
the controller decreases the circumferential speed value of the sending-off roll, and
the tension-adjusting device changes the circumferential speed value of the driven roll,
the changing being performed based on an amount of change in a direction in which the circumferential speed value decreases based on the detection signal and based on the value outputted from the second sensor.

10. A method for manufacturing a sheet-like member associated with an absorbent article according to any one of claims 1 to 9, wherein
the feeding device is at least two feeding devices that feed the second sheet from the sheet roll,
an accumulating device is provided at a position in the direction of transport between the feeding devices and the sending-off device,
the accumulating device being capable of accumulating the second sheet in a form of loop,
when, in order to switch the feeding operation of the second sheet from one feeding device of the two feeding devices to another feeding device of the two feeding devices, the feeding operation being performed by the one feeding device is brought to stop,
the accumulating device makes the loop smaller, to supply the second sheet to the sending-off device,
a second sheet whose feeding operation in the one feeding device is stopped is joined to a second sheet of a sheet roll supported by the other feeding device,
after the joining and after the other feeding device starts a feeding operation of the second sheet from the sheet roll, the accumulating device makes the loop larger, to accumulate the second sheet fed by the other feeding device,
the accumulating device includes:
a plurality of fixed rollers arranged at predetermined positions;
a movable roller guided so as to reciprocate in a direction in which a size of the loop of the second sheet can change with respect to the fixed rollers;
an actuator that reciprocates the movable roller in a direction of movement,
the direction of movement being the direction in which the size of the loop of the second sheet can change, and
the accumulating device forms the loop by wrapping the second sheet around the fixed roller and the movable roller,
when the actuator moves the movable roller in a direction in which the movable roller approaches the fixed roller,
the loop becomes smaller, to supply the accumulated second sheet to the sending-off device,
when the actuator moves the movable roller in a direction in which the movable roller is away from the fixed roller,
the loop becomes large to a predetermined length, to accumulate the second sheet,
a period until a switching of the feeding operation includes a period during which the actuator holds the movable roller so that the movable roller stays at a predetermined position in the direction of movement.

11. A method for manufacturing a sheet-like member associated with an absorbent article according to claim 10, wherein
when making the loop smaller,
the actuator is controlled so that a tension-related value indicated by the value of the first sensor approaches a predetermined third target value.

12. A method for manufacturing a sheet-like member associated with an absorbent article according to claim 10 or 11, wherein
when, in order to make the loop larger, the actuator moves the movable roller to a position that corresponds to the predetermined length,
the feeding operation is performed by the other feeding device so that a tension-related value indicated by the value of the first sensor approaches a predetermined fourth target value.

13. A method for manufacturing a sheet-like member associated with an absorbent article according to any one of claims 10 to 12, wherein
when making the loop smaller,
the actuator moves the movable roller in the direction of movement toward a direction in which the loop becomes small, by applying to the movable roller a force in the direction,
when making the loop larger,
the actuator moves the movable roller in the direction of movement toward a direction in which the loop becomes large, by applying to the movable roller a force in the direction.

14. A method for manufacturing a sheet-like member associated with an absorbent article according to any one of claims 1 to 9, wherein
the feeding device is at least two feeding devices that feed the second sheet from the sheet roll,
when switching the feeding operation from one feeding device of the two feeding devices to another feeding device of the two feeding devices,
after detecting that a circumferential speed value of a sheet roll supported by the other feeding device increases to a same value as a feeding velocity of a second sheet in the one feeding device,
a second sheet of the sheet roll supported by the other feeding device is joined to the second sheet fed by the one feeding device.

15. A method for manufacturing a sheet-like member associated with an absorbent article according to any one of claims 10 to 14, wherein
concerning the following two positions:
a predetermined position on a display item of the second sheet fed by the one feeding device; and
a position that is on a display item of the second sheet of the sheet roll supported by the other feeding device and that corresponds to the predetermined position,
when switching the feeding operation from the one feeding device to the other feeding device,
the second sheet of the sheet roll supported by the other feeding device is joined to the second sheet fed by the one feeding device while positioning so that a displacement amount in the direction of transport between the predetermined position and the position is within ±5% of the second pitch of the display item.

16. A device for manufacturing a sheet-like member associated with an absorbent article,
the manufacturing being performed by stacking and fixing a continuous first sheet and a continuous second sheet while aligning a direction in which the first sheet continues with a direction in which the second sheet continues,
the first sheet having a predetermined first pitch in the direction in which the first sheet continues,
the first pitch being associated with a single piece of the absorbent article,
the second sheet having a display item at a second pitch in the direction in which the second sheet continues,
the second pitch being smaller than the first pitch,
the display item being formed before the second sheet becomes in a state of a sheet roll by being wound in the direction in which the second sheet continues,
the device comprising:
a feeding device that feeds the second sheet from the sheet roll;
a sending-off device that sends-off the second sheet toward a merging position in a transportation path of the first sheet,
the second sheet being fed and transported in in a direction of transport,
the direction of transport being the direction in which the second sheet continues;
three rolls that are rotatably supported at a predetermined position in the direction of transport, the predetermined position being between the feeding device and the sending-off device; and
a first sensor that outputs a value,
the value depending on force that is applied to a center roll of three rolls by the second sheet wrapped around the three rolls,
based on the value, the feeding device performing feeding operation of the second sheet from the sheet roll,
while sending-off the second sheet, the sending-off device changing a stretching state of the second sheet in the direction of transport
so that the display item of the second sheet being transported in the direction of transport is aligned at the first pitch, and
so that the display item is located at a target position on the first sheet that is determined based on the first pitch and where the display item is to be placed.
